# EUROPEAN PATENT APPLICATION

(11) **EP 1 500 662 A2**
(43) Date of publication of application: **26.01.2005**
(21) Application number: 04011908.3
(22) Date of filing: 19.05.2004
(51) Int. Cl.: C07K 14/16, C12N 15/63, A61K 38/16

(54) **Stabilizing peptides**

(30) Priority: 23.05.2003 US; 05.08.2003 US 492704 P
(71) Applicant: CORNELL RESEARCH FOUNDATION, INC., Ithaca, NY 14850 (US)
(72) Inventor: Lu, Min, New York, New York 10021 (US)
(74) Representative: Gardner, Rebecca

(57) **Abstract**

The invention provides peptide inhibitors of HIV infection and methods for stabilizing those peptides. More specifically, it provides a stabilizing peptide which when linked to a second peptide or polypeptide confers increased stability to said second peptide or polypeptide, wherein said stabilizing peptide comprises any one of the amino acid sequences of SEQ ID NOs: 10, 11, 12, or 13 or variants or derivatives thereof.

## Description

### Government Funding

The invention described in this application was made with funds from the National Institute of Health, Grant Numbers NIH AI42382 and AI51151. The United States government has certain rights in the invention.

### Field of the Invention

The invention relates to novel peptides for stabilizing peptides or polypeptides. Such stabilizing peptides can be fused to peptides that inhibit viral fusion with mammalian cellular membranes, thereby allowing such viral peptide inhibitors to be readily prepared by recombinant procedures.

### Background of the Invention

Although the HIV-1 epidemic would be most efficiently controlled by the development of a prophylactic vaccine, no vaccine in clinical development appears close to general public availability. In the absence of treatment, the vast majority of infected individuals will die of AIDS. Moreover, while anti -HIV-1 combination drug therapies have markedly reduced AIDS death rates and have improved the health of many patients in industrialized countries, effective antiviral treatment is not economically feasible at the present time for those populations most in need. The success of protease and reverse transcriptase inhibitors is also tempered by the incidence of toxic side effects, long-term adverse reactions, and the emergence of drug-resistant HIV-1 variants (Little et al., 2002). Hence, new approaches to the treatment and prevention of HIV-1 infection are needed.

Moreover, prevention of infection by a variety of viral types would circumvent the need for expensive and often unsuccessful viral treatment regimens. Clearly, additional agents for antiviral intervention are needed.

### Summary of the Invention

The present invention is directed to stabilizing peptides that, when fused to peptidyl or polypeptide sequences, can stabilize those peptidyl or polypeptide sequences. Such stabilizing peptides have a variety of uses, including fusion to peptides that are produced recombinantly and/or used for *in vivo* therapeutic purposes. For example, peptides having sequences relating to viral glycoproteins that mediate viral fusion can be used to prevent viral entry. While some such peptides are currently available, they are generally unstable and must be made by expensive chemical synthetic procedures. However, the invention provides stabilizing peptides and methods that can dramatically increase the stability of such therapeutic peptides, thereby increasing the *in vivo* half life of such peptides and permitting those peptides to be made recombinantly.

Hence, in one embodiment, the invention provides a stabilizing peptide comprising any one of amino acid sequences SEQ ID NO:10, 11, 12, or 13. Such stabilizing peptides can be fused or linked to any selected peptide in order to increase the stability of the selected peptide and/or to permit the selected to be made recombinantly.

The invention is further directed to stabilizing peptides that can enhance the propensity of a second peptide or polypeptide to fold into an α-helix, wherein the stabilizing peptides comprise any one of amino acid sequences SEQ ID NO:10, 11, 12, or 13.

The degree of α-helicity may conveniently be measured by Circular Dichroism Spectroscopy, for example as described in the Example herein.

The invention is also directed to a composition that includes a carrier and a stabilizing peptide that can enhance the propensity of a second peptide or polypeptide to fold into an α-helix, wherein the stabilizing peptide comprises any one of amino acid sequences SEQ ID NO:10, 11, 12, or 13.

The invention is further directed to a method of making a stable fusion peptide which method comprises expressing in a host cell a chimeric peptide comprising a selected peptide and a stabilizing peptide, wherein the stabilizing peptide comprises any one of amino acid sequences SEQ ID NO:10, 11, 12, or 13. One useful host cell for expression of the peptides of the invention is a bacterial host cell. While host cell proteases will generally digest small peptides, linkage to the stabilizing peptides of the invention generates chimeric peptides that are sufficiently stable to be isolated intact from the host cell. Isolation can be by available procedures, for example, by isolating inclusion bodies and then purifying the chimeric peptide therefrom.

In another embodiment, peptides having sequences related to a gp41 HR2 region are useful for inhibiting fusion of HIV with mammalian cells. These peptides inhibit the fusion of many strains, isolates and types of HIV. However, without modification, such gp41 HR2 peptides tend to be too unstable to be recombinantly prepared or to be widely useful as therapeutic agents. Hence, the invention provides improved peptide inhibitors with sequences that are modified to enhance their stability. Moreover, the invention also provides stabilizing peptides that can be linked to the gp41 HR2 peptides to further increase their stability. Peptides of the invention can be made recombinantly and are useful therapeutic agents for inhibiting HIV infection.

The invention is therefore directed to peptide inhibitors that can inhibit fusion of a human immunodeficiency virus, wherein the peptide inhibitor is linked to a stabilizing peptide that can enhance the propensity of the peptide inhibitor to fold into an α-helix. Examples of stabilizing peptide useful for this purpose include peptide having any one of amino acid sequences SEQ ID NO:10, 11, 12, or 13. The peptide inhibitor can include, for example, a peptide having any one of amino acid sequences SEQ ID NO:7, 8, or 9.

The invention is also directed to stable peptide inhibitor that can inhibit fusion of a human immunodeficiency virus with mammalian cells, wherein the peptide inhibitor comprises any one of amino acid sequences SEQ ID NO:14, 15, 16, 17, or 18.

The peptide inhibitor can bind to an envelope glycoprotein from a human immunodeficiency virus, for example, to a gp41 subunit of the envelope glycoprotein. In some embodiments, the peptide can bind to a coiled coil of a prehairpin intermediate formed by the gp41 subunit of the envelope glycoprotein.

The invention is also directed to peptide comprising any one of amino acid sequences SEQ ID NO:8, 9, 14, 15, 16, 17, or 18. The invention is further directed to a composition comprising a carrier and a peptide inhibitor comprising any one of amino acid sequences SEQ ID NO:8, 9, 14, 15, 16, 17, or 18.

The invention is also directed to a composition including a carrier and a peptide inhibitor that can inhibit fusion of a human immunodeficiency virus, wherein the peptide inhibitor is linked to a stabilizing peptide that can enhance the propensity of the peptide inhibitor to fold into an α-helix, wherein the stabilizing peptide comprises any one of amino acid sequences SEQ ID NO:10, 11, 12, or 13. The peptide inhibitor can include, for example, any one of amino acid sequences SEQ ID NO:7, 8, or 9.

The invention is further directed to a composition comprising a carrier and a stable peptide inhibitor that can inhibit fusion of a human immunodeficiency virus with mammalian cells, wherein the peptide inhibitor comprises any one of amino acid sequences SEQ ID NO:14, 15, 16, 17, or 18.

The invention is also directed to a method of preventing or treating infection by human immunodeficiency virus in a mammal that includes administering to the mammal an effective amount of a peptide inhibitor comprising any one of amino acid sequences SEQ ID NO:8, or 9.

The invention is further directed to a method of preventing or treating infection by human immunodeficiency virus in a mammal comprising administering to the mammal an effective amount of a peptide inhibitor comprising any one of amino acid sequences SEQ ID NO:8, 9, 14, 15, 16, 17, or 18.

The invention is further directed to a method of preventing or treating infection by human immunodeficiency virus in a mammal that includes administering to the mammal an effective amount of a peptide inhibitor linked to a stabilizing peptide that can enhance the propensity of the peptide inhibitor to fold into an α-helix, wherein the stabilizing peptide comprises any one of amino acid sequences SEQ ID NO:10, 11, 12, or 13. The peptide inhibitor can include any one of amino acid sequences SEQ ID NO:7, 8, or 9.

The invention is also directed to a method of making a stable peptide inhibitor that can inhibit fusion of a human immunodeficiency virus with mammalian cells, which method comprises expressing in a host cell a peptide inhibitor comprising any one of amino acid sequences SEQ ID NO:7, 8, 9, 14, 15, 16, 17, or 18. One useful host cell for expression of the peptides of the invention is a bacterial host cell. While the host cell proteases will generally digest small peptides, the peptide inhibitors of the invention are sufficiently stable to be isolated intact from the host cell. Isolation can be by available procedures, for example, by isolating inclusion bodies and then purifying the peptide inhibitor therefrom.

### Brief Description of the Drawings

FIG. 1 provides amino acid sequences of recombinant polypeptide chimera of HIV-1 gp41 (residues 624-675) and GCN4-pII (residues 1-17). A continuous helix is assumed between the gp41 HR2 region and GCN4-pII. The locations of Ile-646→Phe, Gln-652→Leu, Asn-656→Ala, and Leu-661→Phe substitutions in the gp41 HR2 region are shown, using gp160 numbering of the HIV-1 HXB2 strain.
FIG. 2 illustrates that the C52A peptide forms an α-helical structure. (a) Circular dichroism spectrum of C52A (10 µM) in 50 mM Tris-HCl (pH 8.0) and 10 mM NaCl at 4°C. (b) Thermal denaturation of C52A monitored by ellipticity at 222 nm.
FIG. 3 illustrates that the C52C peptide forms an α-helical structure. (a) Circular dichroism spectrum of C52C (10 µM) in 50 mM Tris-HCl (pH 8.0) and 10 mM NaCl at 4°C. (b) Thermal denaturation of C52C monitored by ellipticity at 222 nm.
FIG. 4 illustrates that the C52D peptide forms an α-helical structure. (a) Circular dichroism spectrum of C52D (10 µM) in 50 mM Tris-HCl (pH 8.0) and 10 mM NaCl at 4°C. (b) Thermal denaturation of C52D monitored by ellipticity at 222 nm.
FIG. 5 illustrates that the C52F peptide forms an α-helical structure. (a) Circular dichroism spectrum of C52F (10 µM) in 50 mM Tris-HCl (pH 8.0) and 10 mM NaCl at 4°C. (b) Thermal denaturation of C52F monitored by ellipticity at 222 nm.
FIG. 6 illustrates that the C52L peptide forms an α-helical structure. (a) Circular dichroism spectrum of C52L (10 µM) in 50 mM Tris-HCl (pH 8.0) and 10 mM NaCl at 4°C. (b) Thermal denaturation of C52L monitored by ellipticity at 222 nm.

### Detailed Description of the Invention

The invention provides stabilizing peptides that can be linked to a selected peptide or polypeptide and improve the stability of the selected peptide relative to an unlinked peptide. These fusion peptides can be produced recombinantly, for example, in bacterial host cells. In one embodiment, the stabilizing peptides of the invention can be fused to viral fusion inhibitors that inhibit membrane fusion between mammalian cells and viral particles. Such peptide inhibitors can, for example, inhibit the entry of HIV into mammalian cells.

### Definitions

As used herein, a "derivative" peptide is one whose amino acid sequence is not identical to the reference peptide and, while possessing functionally similar inhibitory properties relative to the reference peptide, the derivative peptide has additional features or amino acids that provide at least one useful property. For example, derivative peptides include peptides that have increased stability, resistance to proteolytic activity, improved pharmacokinetics or that are modified with chemical labels to facilitate detection. Derivative peptides can also include branched and cyclized peptides.

As used herein, human immunodeficiency virus (HIV) includes but is not limited to type 1 (HIV-1), which is the human immunodeficiency virus type-1. HIV-1 includes but is not limited to extracellular virus particles and the forms of HIV-1 found in HIV-1 infected cells.

As used herein, "HIV-1 infection" means the introduction of HIV-1 genetic information into a target cell, such as by fusion of the target cell membrane with HIV-1 or with a cell expressing an HIV-1 envelope glycoprotein⁺. The target cell may be a cell within a mammal that is or may become infected with HIV-1. In some embodiments, the target cell is a cell within a human.

As used herein, "inhibiting HIV-1 infection" means the reduction of the amount of HIV-1 genetic information introduced into a target cell population as compared to the amount that would be introduced without introduction of a selected peptide inhibitor.

As used herein, "peptide" and "polypeptide" are used interchangeably to denote two or more amino acids linked by a peptidyl bond between the α-carboxyl group of one amino acid and the α-amino group of the next amino acid. Peptides may be produced by solid-phase synthetic methods that are well-known to those skilled in the art. In addition to the twenty natural amino acids that are used for protein synthesis *in vivo,* peptides may contain additional amino acids, including but not limited to hydroxyproline, sarcosine, and γ-carboxyglutamate. The peptides may contain modifying groups including but not limited to sulfate and phosphate moieties. Peptides can be comprised of L-or D-amino acids, which are mirror-image forms with differing optical properties. Peptides containing D-amino acids have the advantage of being less susceptible to proteolysis *in vivo.*

This invention provides variants of the peptides described herein. As used herein, a "variant" peptide is one whose amino acid sequence is not identical to the reference peptide but which possesses functionally similar binding properties. Variant peptides may contain N-terminal, C-terminal and/or internal insertions, deletions, or substitutions of amino acids, with the proviso that such insertions, deletions and substitutions do not abrogate the anti-viral properties of the peptide.

### Stabilizing Peptides

According to the present invention, peptides that are fused to stabilizing peptides of the invention have increased stability. Such increased stability permits recombinant production of peptides that would otherwise be too unstable to be produced and/or that otherwise would be too unstable to be widely useful as therapeutic agents. Hence, the stabilizing peptides can be attached or recombinantly fused to a selected peptide to improve the stability of that selected peptide and permit less expensive and simpler methods for recombinant production of the selected peptide.

While not wishing to be bound to any particular theory or mechanism, it is believed that the stabilizing peptides of the invention encourage a selected peptide to form an α-helix, thereby increasing the stability of the selected peptide in solution and providing it with better pharmacodynamics *in vivo.* For example, stabilized peptides of the invention have an increased melting temperature *in vitro* and/or an increased half-life *in vivo.*

Thus, the invention provides peptidyl sequences that can be linked to any peptide selected by one of skill in the art to further enhance the stability and pharmacodynamic properties of the selected peptide *in vivo.* These peptidyl sequences are referred to herein as "stabilizing peptides." The stabilizing peptides of the invention include any peptide that can form an α-helix or that, when linked to another peptide, confers increased stability to that peptide, and that has KIK (SEQ ID NO:10).

Examples of sequences for stabilizing peptides of the invention are as follows:

KIKQIEDKIK (SEQ ID NO:11)

KIKQIEDKIEEEIK (SEQ ID NO: 12)

KIKQIEDKIEEEIKSKIK (SEQ ID NO:13)

In one embodiment, the stabilizing peptides of the invention are used to stabilize peptide inhibitors of viral entry into mammalian cells.

### Viral Entry

Retroviruses are a family of RNA viruses that infect cells in a two step mechanism. These viruses contain two envelope glycoprotein subunits sometimes designated as the surface and transmembrane glycoproteins. The surface and transmembrane glycoproteins form an oligomeric complex on the viral surface and mediate viral entry. The surface glycoprotein contains the viral receptor binding determinants whereas the transmembrane protein contains a hydrophobic transmembrane region and a separate hydrophobic segment that mediates virus-cell membrane fusion. Weiss, R. A., "Cellular receptors and viral glycoproteins involved in retrovirus entry," pp. 1-107, in J. A. Levy (ed.), The Retroviridae, Vol. 2., Plenum Press: New York, N.Y. (1993).

The first step of infection is the binding of the viral particle via the surface protein of the retrovirus envelope (env) protein and viral and cellular membrane fusion for viral uptake via the transmembrane protein of the env protein. The env protein is largely responsible for the specificity (between cell-types and between species) of the infectivity of retroviruses.

Adenoviruses have a linear double-stranded DNA genome. Adenoviruses infect cells by a two step mechanism. First, a viral surface fiber protein binds specifically to a cell surface receptor. In the case of human HeLa cells, the receptor for adenoviruses 2 and 5 is designated CAR, a member of the immunoglobulin protein superfamily, which also serves as a cellular receptor for coxsackie B viruses (Bergelson, Science, 275:1320-1323 (1996)). However, other viral receptors have been described. Following receptor binding, adenoviruses are taken up into the cell by receptor-mediated endocytosis and interaction between the viral penton base protein and cellular integrins is necessary for viral entry. Wickham, Cell, 73:309 (1993); Bai, J. Virol., 68:5925 (1994); Goldman, J. Virol., 69:5951 (1995); and Huang, J. Virol., 70:4502 (1996). The viral DNA is replicated in the cell extrachromosomally. Horwitz, M. S., "Adenoviruses," in Fields Virology, Third Edition, edited by B. N. Fields, D. M. Knipe, P. M. Howley et al., Lippincott-Raven Publishers: Philadelphia, Pa. (1996).

Adeno-associated viruses (AAV) have a linear single-stranded DNA genome and their receptor has not yet been described. These viruses only undergo productive infection if the infected cells are coinfected with a helper virus (e.g., adeno- or herpesvirus) otherwise the genome becomes integrated in a latent state at a specific site on a human chromosome (Linden, Proc. Natl. Acad. Sci. USA, 93:11288-11294 (1996); and Bems, K. J., "Parvoviridae: The viruses and their replication" in Fields Virology, Third Edition, edited by B. N. Fields, D. M. Knipe, P. M. Howley et al., Lippincott-Raven Publishers: Philadelphia, Pa. (1996)). Recombinant adeno-associated viruses are typically made by replacing viral genes with desired genes of interest or by simply adding the terminal AAV DNA sequences (ITRs) to these genes.

Negative strand RNA viruses infect cells by a variety of different mechanisms. For example, Influenza A viruses, which have a segmented RNA genome, contain a surface hemaglutinin protein that binds to cell surface sialic acid receptors and mediates viral entry in a low pH endosome following receptor-mediated endocytosis. Lamb, R. A. and Krug, R. M., "Orthomyxoviridae: The viruses and their replication" Fields Virology, Third Edition, edited by B. N. Fields, D. M. Knipe, P. M. Howley et al., Lippincott - Raven Publishers: Philadelphia, Pa. (1996).

Paramyxoviruses which have a non-segmented RNA genome have two surface viral proteins, the hemaglutinin (HN) and fusion protein (F), required for viral entry which occurs at neutral pH. These viruses can utilize sialic acid receptors, or protein receptors (e.g., CD46 used by measles virus), for viral entry. Lamb, R. A. and Kolakofsky, D., "Paramyxoviridae: The viruses and their replication" Fields Virology, Third Edition, edited by B. N. Fields, D. M. Knipe, P. M. Howley et al., Lippincott-Raven Publishers: Philadelphia, Pa (1996).

Rhabdoviruses (e.g., VSV) which have a non-segmented RNA genome, contain a surface protein (G) that binds to specific cell surface receptors and mediates viral entry in a low pH endosome. A specific phospholipid appears to be one of the receptors for VSV. Wagner, R. R. and Rose, J. K., in Fields Virology, Third Edition, edited by B. N. Fields, D. M. Knipe, P. M. Howley et al., Lippincott-Raven Publishers: Philadelphia, Pa. (1996).

According to the invention, a peptide inhibitor that can decrease entry of any virus can be stabilized by fusion of the peptide inhibitor to a stabilizing peptide of the invention. Such stabilizing peptides can have amino acid sequences such as SEQ ID NO:10, 11, 12, or 13.

Entry of the human immunodeficiency virus type 1 (HIV-1) into mammalian cells is mediated by its envelope glycoprotein. This envelope protein consists of two non-covalently associated subunits, gp120 and gp41, that are generated by proteolytic cleavage of a precursor polypeptide, gp160. Luciw, P. A., in B. N. Fields et al., eds., FIELDS VIROLOGY 1881-1952 (3^{rd} Edition, Lippincott-Raven Publishers, Philadelphia 1996); Freed, E. O. et al., J. BIOL. CHEM. 270: 23883-86 (1995).

Many examples of nucleotide and amino acids for gp160 sequences are available, for example, in the database provided by the National Center for Biotechnology Information (NCBI) (see http://www.ncbi.nlm.nih.gov/). One example of a sequence for gp160 is the amino acid sequence at NCBI accession number AAA76668 (gi: 665491); a nucleotide sequence for this gp160 protein is available at accession number U12032 (gi: 665490). *See* website at ncbi.nlm.nih.gov. The amino acid sequence for this gp160 protein is provided below (SEQ ID NO:1).

1 MRVKEKYQHL RRWGWRWGTM LLGMLMICSA TEKLWVTVYY
41 GVPVWKEATT TLFCASDAKA YDTEVHNVWA THACVPTDPN
81 PQEVVLVNVT ENFNMWKNDM VEQMHEDIIS LWDQSLKPCV
121 KLTPLCVSLK CTDLKNDTNT NSSSGGMIME KGEIKNCSFN
161 ISTSIRGKVQ KEYAFFYKLD IIPIDNDTTS YTLTSCNTSV
201 ITQACPKVSF EPIPIHYCAP AGFAILKCNN KTFNGTGPCT
241 NVSTVQCTHG IRPVVSTQLL LNGSLAEEEV VIRSANFTDN
281 VKTIIVQLNQ SVEINCTKPN NNTGKRIRIQ RGPGRTFVTI
321 GKIGNMRQAH CNISRAKWNN TLKQIASKLR EQYGNNKTII
361 FKQSSGGDLE IVTHSFNCGG EFFYCNSTQL FNSTWFNSTG
401 SNNTEGSDTI TLPCRIKQII NMWQEVGKAM YAPPISGQIR
441 CSSNITGLLL TRDGGNNNNG SEIFRPGGGD MRDNWRSELY
481 KYKVVKIEPL GVAPTKAKRR VVQREKRAVG IGALFLGFLG
521 AAGSTMGAAS MTLTVQARQL LSGIVQQQNN LLRAIEAQQH
561 LLQLTVWGIK QLQARILAVE RYLKDQQLLG IWGCSGKLIC
601 TTAVPWNASW SNKSLERIWN HTTWMEWDRE INNYTSLIHS
641 LIEESQNQQE KNEQELLELD KWASLWNWFN ITNWLWYVKI
681 FIMIVGGLVG LRIVFAVLSI VNRVRQGYSP LSFQTHLPTP
721 GGPDRPEGIE EEGGERDRDR SIRLVNGS

Another example of an amino acid sequence for a HIV gp160 protein is available in the NCBI database at accession number AAA76666 (gi: 665487); the nucleotide sequence for this HIV gp160 protein can be found at accession number U12030 (gi: 665486). See website at ncbi.nlm.nih.gov. Many more sequences for HIV gp160 are available, for example, at the ncbi.nlm.nih.gov website.

The gp120 protein derived from the gp160 precursor directs target -cell recognition and viral tropism through interaction with the cell-surface receptor CD4 and one of several co-receptors that are members of the chemokine receptor family. Broder, C. C. et al., PATHOBIOLOGY 64:171 -179 (1996); D'Souza, M. P. et al., NATURE MED. 2:1293-1300 (1996); Wilkinson, D., CURRENT BIOLOGY 6:1051-1053 (1996). The membrane-spanning gp41 subunit then promotes fusion of the viral and cellular membranes, a process that results in the release of viral contents into the host cell.

Binding of gp120/gp41 complexes to cellular receptors (e.g. CD4 and a chemokine receptor such as CCR5 or CXCR4) triggers a series of structural rearrangements in the envelope glycoprotein. A transient species arises, termed the prehairpin intermediate, in which gp41 exists as a membrane protein simultaneously in both the viral and cellular membranes. This extended gp41 prehairpin intermediate ultimately collapses into a trimer-of-hairpins structure that provides sufficient tension to drive membrane fusion. The core of the HIV - 1 trimer-of-hairpins is a bundle of six α-helices from three gp41 ectodomains. Three α-helices derived from the N-terminal HR1 regions form a central, trimeric coiled coil, around which three α-helices derived from the C-terminal HR2 regions pack in an anti-parallel manner into hydrophobic grooves on the surface of the coiled coil. Thus, formation of the timer-of-hairpins structure is believed to bring the membranes into close apposition necessary for the fusion event.

The gp120 and gp41 envelope glycoproteins can, of course, have a variety of sequences, depending upon the strain or type of HIV. One example of a sequence for the envelope glycoprotein complex (gp120/gp41) is the amino acid sequence at accession number S21998 (gi: 94245). See website at ncbi.nlm.nih.gov. The amino acid sequence for this gp120/gp41 complex is provided below (SEQ ID NO:2). 
1 KAKRRVVQRE KRAVGMGAAF FLGFLGAAGS TMGAASLTLT

41 VQARLLLSGI VQQQNNLLRA IEAHEHLLQL TVWGIKQLQA

81 RILAVERYLK DQQLLGIWGC SGKLICTTTV PWNASWSNKS

121 LDKIWNNMTW MEWDREINNY TSLIYTLIEQ SQNQQEKNEQ

161 ELLELDKWAS LWNWFDITQW LWYIKIFIMI VGGLIGLRIV

201 FTVLSIVNRV RQGYSPLSFQ TRRPARRGPD RPEGIEEEGG

241 ERDRDRSGRL VNGFLALIWD DLRSLCLFSY HRLRDLLLIV
 
281 TRIVELLGRR GWEVLKYLWN LLQYWSQELK NSAVSLLNAT
321 AIAVAEGTDR VIELLQRAFR AILHIPRRXR QGLERALL
 Many more sequences for HIV gp120/gp41 complexes are available, for example, at the ncbi.nlm.nih.gov website.

One example of a sequence for a gp41 subunit is the amino acid sequence at accession number AAM51938 (gi: 31280834). See website at ncbi.nlm.nih.gov. The amino acid sequence for this gp41 polypeptide is provided below (SEQ ID NO:3).

1 AQQHLLRLTV WGIKQLQARV LALERYLKDQ QLLGIWGCSG
41 RLICTTNVPW NSSWSNKTYN DIWDNMTWLQ WDKEISNYTN
81 IIYTLIEESQ NQQEKNEQDL LALDKWASLW SWFDITN

Another example of a sequence for a gp41 subunit is the amino acid sequence at accession number AAM51921 (gi: 31280800). See website at ncbi.nlm.nih.gov. The amino acid sequence for this gp41 polypeptide is provided below (SEQ ID NO:4).

1 RSQHLLKLTV WGIKQLQARV LALERYLKDQ QLLGIWGCSG
41 KLICTTNVPW NSSWSNKTYE YIWGNMTWLQ WEKEIDNYTS
81 LIYTLIEESQ IQQEKNEQDL LALDEWASLW SWFSITK

HIV can undergo mutation to give rise to altered glycoprotein sequences. In the HXB2 isolate, for example, the gp160 envelope glycoprotein can have the amino acid sequence at accession number P04578 (gi: 6015102). See website at ncbi.nlm.nih.gov. The amino acid sequence for this gp160 polypeptide is provided below (SEQ ID NO:5).

1 MRVKEKYQHL WRWGWRWGTM LLGMLMICSA TEKLWVTVYY
41 GVPVWKEATT TLFCASDAKA YDTEVHNVWA THACVPTDPN
81 PQEVVLVNVT ENFNMWKNDM VEQMHEDIIS LWDQSLKPCV
121 KLTPLCVSLK CTDLKNDTNT NSSSGRMIME KGEIKNCSFN
161 ISTSIRGKVQ KEYAFFYKLD IIPIDNDTTS YKLTSCNTSV
201 ITQACPKVSF EPIPIHYCAP AGFAILKCNN KTFNGTGPCT
241 NVSTVQCTHG IRPVVSTQLL LNGSLAEEEV VIRSVNFTDN

281 AKTIIVQLNT SVEINCTRPN NNTRKRIRIQ RGPGRAFVTI
321 GKIGNMRQAH CNISRAKWNN TLKQIASKLR EQFGNNKTII
361 FKQSSGGDPE IVTHSFNCGG EFFYCNSTQL FNSTWFNSTW
401 STEGSNNTEG SDTITLPCRI KQIINMWQKV GKAMYAPPIS
441 GQIRCSSNIT GLLLTRDGGN SNNESEIFRP GGGDMRDNWR
481 SELYKYKVVK IEPLGVAPTK AKRRVVQREK RAVGIGALFL
521 GFLGAAGSTM GAASMTLTVQ ARQLLSGIVQ QQNNLLRAIE
561 AQQHLLQLTV WGIKQLQARI LAVERYLKDQ QLLGIWGCSG
601 KLICTTAVPW NASWSNKSLE QIWNHTTWME WDREINNYTS
641 LIHSLIEESQ NQQEKNEQEL LELDKWASLW NWFNITNWLW
681 YIKLFIMIVG GLVGLRIVFA VLSIVNRVRQ GYSPLSFQTH
721 LPTPRGPDRP EGIEEEGGER DRDRSIRLVN GSLALIWDDL
761 RSLCLFSYHR LRDLLLIVTR IVELLGRRGW EALKYWWNLL
801 QYWSQELKNS AVSLLNATAI AVAEGTDRVI EVVQGACRAI
841 RHIPRRIRQG LERILL

Synthetic peptides called C peptides (e.g., the T-20 peptide) that are derived from the gp41 HR2 region (e.g. at about positions 638-673 of SEQ ID NO:5) have been observed to inhibit HIV-1 entry (Jiang et al., 1993; Wild et al., 1994). The sequence of the T-20 peptide is provided below (SEQ ID NO:6).

Acetyl-YTSLIHSLIE ESQNQQEKNE QELLELDKWA SLWNWF

Some evidence indicates that C peptides act in a dominant-negative manner by binding the HR1 coiled coil of the prehairpin intermediate and preventing formation of the trimer-of hairpins, ultimately leading to irreversible loss of membrane-fusion activity (Lu et al., 1995; Wild et al., 1995; Rimsky et al., 1998). The inhibitory activity of C peptides against different HIV isolates may be explained by the conserved nature of a hydrophobic groove on the surface of the HR1 coiled coil to which C peptides bind (Chan et al., 1997; Weissenhorn et al., 1997; Tan et al., 1997).

### Peptide Inhibitors

According to the present invention, peptides having sequences related to a gp41 HR2 region are useful for inhibiting fusion of HIV with mammalian cells. These peptides inhibit the fusion of many strains, isolates and types of HIV. However, without modification, such gp41 HR2 peptides tend to be too unstable to be widely useful as therapeutic agents. Two aspects of the invention are provided to improve the stability and half-life of such peptides: guidance and specific sequence modifications for selecting optimal gp41 peptide inhibitor sequences and added stabilizing peptides that can be attached to the selected gp41 peptide inhibitor to improve the stability of the core peptide inhibitor structure.

In particular, the invention provides gp41 HR2 peptides with sequences that can be modified to enhance their stability. While not wishing to be bound to any particular theory or mechanism, it is believed that peptide inhibitors with an increased tendency to form α-helices, tend to be more stable in solution and to have better pharmacodynamics *in vivo.* For example, stabilized peptide inhibitors of the invention have an increased melting temperature *in vitro* and/or an increased half-life *in vivo.*

In another embodiment, the invention provides peptidyl sequences that can be linked to gp41 HR2 peptides to further enhance their stability and pharmacodynamic properties *in vivo.* These peptides are referred to herein as "stabilizing peptides." Such stabilizing peptides are believed to further improve the ability of the selected gp41 HR2 peptides to form α-helices.

Therefore, in one embodiment, the invention provides gp41 HR2 peptides that can inhibit HIV fusion with mammalian cells. These peptide inhibitors can be modified to promote folding into an α-helical conformation. However, according to the invention, a peptide inhibitor that is too stable may have reduced activity as an inhibitor. Hence, peptide inhibitor sequences are selected to provide excellent inhibitory activity as well as optimized stability.

According to the invention any peptide from the HR2 region of any HIV gp41 subunit can be used in the practice of the invention so long as the peptide can fold into an α-helical conformation and inhibit at least one strain of HIV from fusing with a mammalian cell. Hence, peptides with sequences from any gp41 HR2 region of a HIV envelope protein are contemplated by the invention as inhibitors of HIV fusion, as well as variant or derivative peptides that have one or more amino acids substituted for the amino acids that are naturally present in the selected gp41 peptide. Mixtures of peptides with different sequences are also contemplated.

In general, the peptide sequences, peptide variants and mixtures of peptides are formulated and used in a manner that optimizes inhibition of HIV fusion while providing optimal stability so that the peptide will have an adequate half-life *in vivo.* Hence, the composition and formulations of the present peptides can be varied to increase stability or so that greater levels of inhibition are achieved, HIV infection is prevented or the progression of AIDS is retarded.

The size of a peptide inhibitor can vary. In general, a peptide of only about five to ten amino acids can be too small to provide optimal inhibition. However, peptides of more than about fifteen to twenty amino acids are sufficiently long to provide inhibition. Therefore, while the overall length is not critical, peptides longer than fifteen amino acids are often employed in the invention. Other peptides employed in the invention are longer than twenty amino acids. Still other peptides employed in the invention are longer than thirty amino acids. Moreover, peptides are longer than about forty to forty five amino acids are also used in the invention. In some embodiments the peptides are longer than about fifty amino acids.

There is no particular upper limit on peptide inhibitor size. However, it is generally cheaper to make shorter peptides than longer peptides, and longer peptides may be more immunogenic. Hence, the peptide inhibitors of the invention are generally shorter than about one hundred amino acids. Many peptide inhibitors used in the invention are shorter than about ninety amino acids. Other peptide inhibitors used in the invention are shorter than about eighty five amino acids. Peptides shorter than about seventy five amino acids can also be used. Similarly, peptides shorter than about seventy amino acids are also used in the invention.

Examples of peptides useful for inhibiting HIV entry into mammalian cells have SEQ ID NO:7-9 and 14-18 with about fifty three to seventy acids. Sequences of several representative peptide inhibitors are provided in Table 1, with sequence differences highlighted in bold and with underlining.

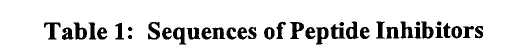

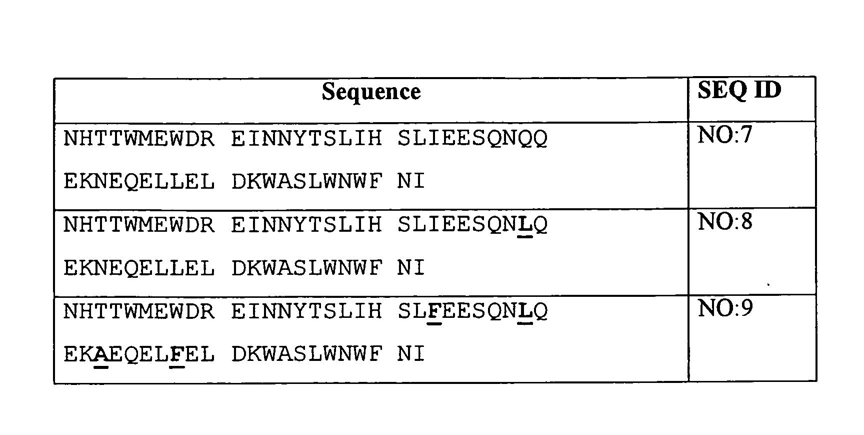

Each of the peptides listed in Table 1, as well as any peptides with amino acid sequences SEQ ID NO:7-9, 14-18 are contemplated as peptide inhibitors of the invention. Peptides with SEQ ID NO:14-18 have stabilizing peptides comprising the amino acid sequence KIK (SEQ ID NO:10) attached at their C-termini. Examples of sequences for stabilizing peptides of the invention are as follows:

KIK (SEQ ID NO:10)

KIKQIEDKIK (SEQ ID NO:11)

KIKQIEDKIEEEIK (SEQ ID NO:12)

KIKQIEDKIEEIKSKIK (SEQ ID NO:13)

As indicated above, stabilizing peptides improve the tendency of peptide inhibitors to fold into an α-helical conformation.

### Peptide Variants and Derivatives

Peptide variants and derivatives of the peptides having any of SEQ ID NO:7-18 are also useful in the invention. Such peptide variants and derivatives can have one or more amino acid substitutions, deletions, insertions or other modifications so long as the peptide variant or derivative can stabilize another peptide or inhibit fusion of HIV with mammalian cells.

Amino acid residues of the isolated peptides can be genetically encoded L-amino acids, naturally occurring non-genetically encoded L-amino acids, synthetic L-amino acids or D-enantiomers of any of the above. The amino acid notations used herein for the twenty genetically encoded L-amino acids and common non-encoded amino acids are conventional and are as shown in Table 2.

**Table 2**

| **Amino Acid** | **One- Letter** Symbol | **Common Abbreviation** |
|---|---|---|
| Alanine | A | Ala |
| Arginine | R | Arg |
| Asparagine | N | Asn |
| Aspartic acid | D | Asp |
| Cysteine | C | Cys |
| Glutamine | Q | Gln |
| Glutamic acid | E | Glu |
| Glycine | G | Gly |
| Histidine | H | His |
| Isoleucine | I | Ile |
| Leucine | L | Leu |
| Lysine | K | Lys |
| Methionine | M | Met |
| Phenylalanine | F | Phe |
| Proline | P | Pro |
| Serine | S | Ser |
| Threonine | T | Thr |
| Tryptophan | W | Trp |
| Tyrosine | Y | Tyr |
| Valine | V | Val |
| β-Alanine | | BAla |
| 2,3-Diaminopropionic acid | | Dpr |
| α-Aminoisobutyric acid | | Aib |
| N-Methylglycine (sarcosine) | | MeGly |
| Ornithine | | Orn |
| Citrulline | | Cit |
| t-Butylalanine | | t-BuA |
| t-Butylglycine | | t-BuG |
| N-methylisoleucine | | MeIle |
| Phenylglycine | | Phg |
| Cyclohexylalanine | | Cha |
| Norleucine | | Nle |
| Naphthylalanine | | Nal |
| Pyridylalanine | | |
| 3-Benzothienyl alanine | | |
| 4-Chlorophenylalanine | | Phe(4-Cl) |
| 2-Fluorophenylalanine | | Phe(2-F) |
| 3-Fluorophenylalanine | | Phe(3-F) |
| 4-Fluorophenylalanine | | Phe(4-F) |
| Penicillamine | | Pen |
| 1,2,3,4-Tetrahydro-isoquinoline-3-carboxylic acid | | Tic |
| β-2-thienylalanine | | Thi |
| Methionine sulfoxide | | MSO |
| Homoarginine | | hArg |
| N-acetyl lysine | | AcLys |
| 2,4-Diamino butyric acid | | Dbu |
| ρ-Aminophenylalanine | | Phe(pNH₂) |
| N-methylvaline | | MeVal |
| Homocysteine | | hCys |
| Homoserine | | hSer |
| ε-Amino hexanoic acid | | Aha |
| δ-Amino valeric acid | | Ava |
| 2,3-Diaminobutyric acid | | Dab |

Peptides that are encompassed within the scope of the invention can have one or more amino acids substituted with an amino acid of similar chemical and/or physical properties, so long as these variant or derivative peptides retain the ability stabilize another peptide or to inhibit fusion of HIV.

Mathematical algorithms, for example, the Smith-Waterman algorithm, can also be used to determine sequence homologies and locate and evaluate variant and derivative peptides. Smith & Waterman, J. Mol. Biol., 147:195 (1981); Pearson, Genomics, 11:635 (1991). Although any sequence algorithm can be used to identify a variant, the present invention generally defines a variant with reference to the Smith-Waterman algorithm, where any one of SEQ ID NOs:7, 8, 9, 10, 11, 12, 13, 14, 15, 16, 17, or 18 is used as the reference sequence to define the percentage of homology of peptide homologues over its length. The choice of parameter values for matches, mismatches, and inserts or deletions is arbitrary, although some parameter values have been found to yield more biologically realistic results than others. One preferred set of parameter values for the Smith-Waterman algorithm is set forth in the "maximum similarity segments" approach, which uses values of 1 for a matched residue and -¹/₃ for a mismatched residue (a residue being either a single nucleotide or single amino acid). Waterman, Bulletin of Mathematical Biology, 46:473 (1984). Insertions and deletions x, are weighted as xₖ = 1 + k/3, where k is the number of residues in a given insertion or deletion. Preferred variant peptides are those having greater than 75% amino acid sequence homology to any one of SEQ ID NOs: 7-18 using the Smith-Waterman algorithm. More preferred variants have greater than 90% amino acid sequence homology. Even more preferred variants have greater than 95% amino acid sequence homology, and most preferred variants have at least 98% amino acid sequence homology.

Amino acids that are substitutable for each other generally reside within similar classes or subclasses. As known to one of skill in the art, amino acids can be placed into three main classes: hydrophilic amino acids, hydrophobic amino acids and cysteine-like amino acids, depending primarily on the characteristics of the amino acid side chain. These main classes may be further divided into subclasses. Hydrophilic amino acids include amino acids having acidic, basic or polar side chains and hydrophobic amino acids include amino acids having aromatic or apolar side chains. Apolar amino acids may be further subdivided to include, among others, aliphatic amino acids. The definitions of the classes of amino acids as used herein are as follows:
"Hydrophobic Amino Acid" refers to an amino acid with a side chain that is uncharged at physiological pH and that is repelled by aqueous solution. Examples of genetically encoded hydrophobic amino acids include Ile, Leu and Val. Examples of non-genetically encoded hydrophobic amino acids include t-BuA.
"Aromatic Amino Acid" refers to a hydrophobic amino acid with a side chain containing at least one ring having a conjugated π-electron system (aromatic group). The aromatic group may be further substituted with substituent groups such as alkyl, alkenyl, alkynyl, hydroxyl, sulfonyl, nitro and amino groups, as well as others. Examples of genetically encoded aromatic amino acids include phenylalanine, tyrosine and tryptophan. Commonly encountered non-genetically encoded aromatic amino acids include phenylglycine, 2-naphthylalanine, β-2-thienylalanine, 1,2,3,4-tetrahydroisoquinoline-3-carboxylic acid, 4-chlorophenylalanine, 2-fluorophenylalanine, 3-fluorophenylalanine and 4-fluorophenylalanine.
"Apolar Amino Acid" refers to a hydrophobic amino acid with a side chain that is generally uncharged at physiological pH and that is not polar. Examples of genetically encoded apolar amino acids include glycine, proline and methionine. Examples of non-encoded apolar amino acids include Cha.
"Aliphatic Amino Acid" refers to an apolar amino acid with a saturated or unsaturated straight chain, branched or cyclic hydrocarbon side chain. Examples of genetically encoded aliphatic amino acids include Ala, Leu, Val and Ile. Examples of non-encoded aliphatic amino acids include Nle.
"Hydrophilic Amino Acid" refers to an amino acid with a side chain that is attracted by aqueous solution. Examples of genetically encoded hydrophilic amino acids include Ser and Lys. Examples of non-encoded hydrophilic amino acids include Cit and hCys.
"Acidic Amino Acid" refers to a hydrophilic amino acid having a side chain pK value of less than 7. Acidic amino acids typically have negatively charged side chains at physiological pH due to loss of a hydrogen ion. Examples of genetically encoded acidic amino acids include aspartic acid (aspartate) and glutamic acid (glutamate).
"Basic Amino Acid" refers to a hydrophilic amino acid having a side chain pK value of greater than 7. Basic amino acids typically have positively charged side chains at physiological pH due to association with hydronium ion. Examples of genetically encoded basic amino acids include arginine, lysine and histidine. Examples of non-genetically encoded basic amino acids include the non-cyclic amino acids omithine, 2,3-diaminopropionic acid, 2,4-diaminobutyric acid and homoarginine.
"Polar Amino Acid" refers to a hydrophilic amino acid having a side chain that is uncharged at physiological pH, but which has a bond in which the pair of electrons shared in common by two atoms is held more closely by one of the atoms. Examples of genetically encoded polar amino acids include asparagine and glutamine. Examples of non-genetically encoded polar amino acids include citrulline, N-acetyl lysine and methionine sulfoxide.
"Cysteine-Like Amino Acid" refers to an amino acid having a side chain capable of forming a covalent linkage with a side chain of another amino acid residue, such as a disulfide linkage. Typically, cysteine-like amino acids generally have a side chain containing at least one thiol (SH) group. Examples of genetically encoded cysteine-like amino acids include cysteine. Examples of non-genetically encoded cysteine-like amino acids include homocysteine and penicillamine.

As will be appreciated by those having skill in the art, the above classifications are not absolute. Several amino acids exhibit more than one characteristic property, and can therefore be included in more than one category. For example, tyrosine has both an aromatic ring and a polar hydroxyl group. Thus, tyrosine has dual properties and can be included in both the aromatic and polar categories. Similarly, in addition to being able to form disulfide linkages, cysteine also has apolar character. Thus, while not strictly classified as a hydrophobic or apolar amino acid, in many instances cysteine can be used to confer hydrophobicity to a peptide.

Certain commonly encountered amino acids that are not genetically encoded and that can be present, or substituted for an amino acid, in the peptides and peptide analogues of the invention include, but are not limited to, β-alanine (b-Ala) and other omega-amino acids such as 3-aminopropionic acid (Dap), 2,3 - diaminopropionic acid (Dpr), 4-aminobutyric acid and so forth; α-aminoisobutyric acid (Aib); ε-aminohexanoic acid (Aha); δ-aminovaleric acid (Ava); methylglycine (MeGly); ornithine (Om); citrulline (Cit); t-butylalanine (t-BuA); t-butylglycine (t-BuG); N-methylisoleucine (MeIle); phenylglycine (Phg); cyclohexylalanine (Cha); norleucine (Nle); 2-naphthylalanine (2-Nal); 4-chlorophenylalanine (Phe(4-Cl)); 2-fluorophenylalanine (Phe(2-F)); 3-fluorophenylalanine (Phe(3-F)); 4-fluorophenylalanine (Phe(4-F)); penicillamine (Pen); 1,2,3,4-tetrahydroisoquinoline-3-carboxylic acid (Tic); β-2-thienylalanine (Thi); methionine sulfoxide (MSO); homoarginine (hArg); N-acetyl lysine (AcLys); 2,3-diaminobutyric acid (Dab); 2,3-diaminobutyric acid (Dbu); p - aminophenylalanine (Phe(pNH₂)); N-methyl valine (MeVal); homocysteine (hCys) and homoserine (hSer). These amino acids also fall into the categories defmed above.

The classifications of the above-described genetically encoded and non-encoded amino acids are summarized in Table 3, below. It is to be understood that Table 3 is for illustrative purposes only and does not purport to be an exhaustive list of amino acid residues that may comprise the peptides and peptide analogues described herein. Other amino acid residues that are useful for making the peptides described herein, as well as variants or derivatives thereof, can be found, for example, in Fasman, 1989, CRC Practical Handbook of Biochemistry and Molecular Biology, CRC Press, Inc., and the references cited therein. Amino acids not specifically mentioned herein can be conveniently classified into the above-described categories on the basis of known behavior and/or their characteristic chemical and/or physical properties as compared with amino acids specifically identified.

**TABLE 3**

| **Classification** | **Genetically Encoded** | **Genetically Non-Encoded** |
|---|---|---|
| Hydrophobic | | |
| Aromatic | F, Y, W | Phg, Nal, Thi, Tic, Phe(4-Cl), Phe(2-F), Phe(3-F), Phe(4-F), Pyridyl Ala, Benzothienyl Ala |
| Apolar | M, G, P | |
| Aliphatic | A, V, L, I | t-BuA, t-BuG, MeIle, Nle, MeVal, Cha, bAla, MeGly, Aib |
| Hydrophilic | | |
| Acidic | D, E | |
| Basic | H, K, R | Dpr, Om, hArg, Phe(p-NH₂), DBU, A₂ BU |
| Polar | Q, N, S, T, Y | Cit, AcLys, MSO, hSer |
| Cysteine-Like | C | Pen, hCys, β-methyl Cys |

Peptides of the invention can have any amino acid substituted by any similarly classified amino acid to create a variant or derivative peptide, so long as the peptide, variant or derivative thereof retains an ability to inhibit HIV fusion.

One of skill in the art can design an appropriate peptide inhibitor, stabilizing peptide or combination of peptide inhibitors/stabilizing peptides to achieve the quality and quantity of inhibition or stability desired using available teachings in combination with the teachings provided herein. "Quality" of inhibition refers to the type, isolate or strain of HIV inhibited. Different HIV strains can have somewhat different envelope proteins and may inhibited to a lesser or a greater extent by a single HIV fusion inhibitor. "Quantity" of inhibition refers to the overall amount of HIV fusion inhibition. By modulating the type and quantity of peptide inhibitor used, the quality and quantity of inhibition can be modulated. Similarly, the "quality" of stability obtained refers to the type of peptide that can be stabilized by a particular stabilizing peptide. The "quantity" of stability refers to the overall amount of stability obtained, for example, the degree to which the melting point of a selected peptide is increased by fusion to a stabilizing peptide of the invention.

Fusion assays for detecting fusion between HIV and mammalian cells can be performed by any of the available procedures for assessing HIV fusion, for example, by a procedure described by Ciminale (AIDS Res. Hum. Retrovir. 1990, 6, 1281-1287). For example, HIV-1 envelope-expressing, Tat-producing HL2/3 cells and CXCR-4 expressing, LTR-βGal-containing MAGI cells (obtained from the AIDS Research and Reference Program, National Institute of Allergy and Infectious Disease, NIH, Bethesda, Md., USA) can be pre-incubated separately with a test peptide for a period of about 1 h at 37 °C, followed by co - incubation of the two cell lines at a cell ratio of about one to one. The cells can then be incubated for about 16 h. To detect fusion, the cells are fixed and stained for the expression of β-gal with indolyl-β-D-galactopyranoside (X-Gal). The number of blue cells (indicating completion of attachment and fusion of membranes) are counted by light microscopy. One of skill in the art can readily make modifications to the peptides provided by the invention and observe the type and degree to which fusion of different HIV strains is inhibited.

Moreover, one of skill in the art can also modify the peptide and observe the effect of that modification on the stability of peptide in solution. The stability of the peptide in solution can be observed by many procedures available to one of skill in the art. For example, the degree to which a peptide folds into an α-helix can be observed by circular dichroism. The stability of this folded structure can be evaluated by determining its melting temperature.

The invention also contemplates modifying the peptide inhibitors to stabilize them, to facilitate their uptake and absorption and to improve any other characteristic or property of the peptides that is known to one of skill in art. For example, charges on the peptide inhibitors can be neutralized, and the peptides can be linked to other chemical moieties. In one embodiment the charges at the N-terminal and C-terminal ends are effectively removed. This can be done by any method available to one of skill in the art, for example, by acetylating the N-terminus and amidating the C-terminus.

Methods for preparing cyclic peptides and modifying peptide in other ways are well-known in the art (see, e.g., Spatola, 1983, Vega Data 1(3) for a general review); Spatola, 1983, "Peptide Backbone Modifications" In: Chemistry and Biochemistry of Amino Acids Peptides and Proteins (Weinstein, ed.), Marcel Dekker, New York, p. 267 (general review); Morley, 1980, Trends Pharm. Sci. 1:463-468; Hudson et al., 1979, Int. J. Prot. Res. 14:177-185 (--CH₂ NH--, -CH₂ CH₂ --); Spatola et al., 1986, Life Sci. 38:1243-1249 (--CH₂ --S); Hann, 1982, J. Chem. Soc. Perkin Trans. I. 1:307-314 (--CH = CH--, cis and trans); Almquist et al., 1980, J. Med. Chem. 23:1392-1398 (--CO CH₂ --); Jennings-White et al., Tetrahedron. Lett. 23:2533 (--CO CH₂ --); European Patent Application EP 45665 (1982) CA:97:39405 (-CH(OH) CH₂ --); Holladay et al., 1983, Tetrahedron Lett. 24:4401-4404 (--C(OH)CH₂--); and Hruby, 1982, Life Sci. 31:189-199 (--CH₂ -S--).

### Therapeutic Methods

It is generally agreed that the development of a safe, effective vaccine to prevent the transmission of HIV-1 is the single most important challenge in AIDS research, as well as one of the most important issues in international public health. Letvin (1998) Science 280, 1875-80; Burton et al. (1998) Nat. Med. 4, 495-98; Bloom et al. (1998) Nat. Med. 4,480-84; Esparza et al. (2000) Lancet 355, 2061-66. Unfortunately, progress in HIV-1 vaccine development has been slow, and it is unlikely that an effective vaccine will be available for widespread use in the immediate future. A different approach is provided by the present invention: inhibition of viral fusion with mammalian cells.

The present invention is directed to methods of preventing or treating HIV infection in a mammal, which include administering to the mammal a therapeutically effective amount of a peptide of the present invention. Preventing infection is intended to include reduction of viral fusion or viral entry into a mammalian host cell. Treatment of, or treating, viral infections is intended to include the alleviation of or diminishment of at least one symptom typically associated with the infection. The treatment also includes alleviation or diminishment of more than one symptom. The treatment may substantially inhibit the spread or progression of the viral infection and/or eliminate the symptoms associated with the infection.

In another embodiment, the invention relates to a vaginal or rectal microbicides, to prevent the sexual transmission of the virus. The case for an accelerated program of microbicide development has been endorsed by many international agencies, including UNAIDS, the World Health Organization, the Population Council and others. American Foundation for AIDS Research. 2000. Microbicides: a new weapon against HIV. AIDS Research Report, AmFAR; European Commission. 1999. A study into the market potential for vaginal microbicides. EU HIV/AIDS Programme in developing countries; UNAIDS 1998. Microbicides for HIV prevention. UNAIDS technical update; The Population Council and International Family Health. 2000. The case for microbicides: A global priority. Moreover, the need for such prevention is highlighted by the continued, dramatic spread of HIV-1 in the countries of the developing world, in which anti-retroviral therapy is mostly unavailable and where the AIDS pandemic has taken its greatest toll. Plot (1998) Science 280, 1844-45; Kaul et al. (2000) Nat. Immunol. 1, 267 -270; Piot (2000) Science 288, 2176-78.

Nearly half of the world's approximately 40 million HIV-1-infected people are women, and current projections are that this proportion will continue to increase. Piot (2000) Science 288, 2176-78; Schwartlander et al. (2000) Science 289, 64-66; Whyte B. (2000). UNAIDS estimates that half the teenagers in some African countries will die of AIDS. Bull World Health Organ 78, 946;. Cock et al. (2000) Trop. Med. Int. Health 5, A3 -9. Over 30 percent of the female populations of sub-Saharan African countries such as Zimbabwe and Botswana, aged thirteen to nineteen, are known to be infected. Among teenagers, the ratio of infected girls to boys is as high as six to one in countries such as Kenya, Tanzania and South Africa. Whether this is because young girls are inherently more susceptible than older women to acquiring HIV-1 is now an active area of research, but such statistics further emphasize the need to protect these young women by the use of an effective vaginal microbicide. Sex-work is common among teen-aged girls, and condom usage is low due to their unavailability and cost of condoms, as well as the ignorance and resistance of customers. The result, all too frequently, is that these girls may simultaneously become virus-infected and pregnant and then give birth to HTV-1 -infected infants nine months later. Garcia-Moreno et al. (2000) Violence against women: its importance for HIV/AIDS. AIDS 14 (supp. 3) S253-S266.

In fact, 1600 infected babies are now bom each day, many to teen-aged mothers. Epidemiologists predict that severe population decline will become a reality in many of the countries of sub-Saharan Africa and Asia as many women of child bearing age are lost to the epidemic. Plot (1998) Science 280, 1844 -45; Kaul et al. (2000) Nat. Immunol. 1, 267-270; Piot (2000) Science 288, 2176-78; Schwartlander et al. (2000) Science 289,64-66; Whyte B. (2000). UNAIDS estimates that half the teenagers in some African countries will die of AIDS. Bull World Health Organ 78, 946; Cock et al. (2000) Trop. Med. Int. Health 5, A3-9.

Barrier contraceptive measures, for example, the use of a latex condom can play an important role in preventing the spread of HIV-1 and other sexually transmitted diseases (STDs). Indeed, the use of condoms may be considered the best alternative to abstinence for preventing the exchange of infectious body fluids during sexual activity. Unfortunately, however, condom usage has a number of practical limitations, including religious and cultural taboos that undermine their use in many parts of the world. All too often, men strongly resist the proposed use of a condom by a commercial or social sex partner who insists on condom usage. Garcia-Moreno et al. (2000) Violence against women: its importance for HIV/AIDS. AIDS 14 (supp. 3) S253-S266. These reasons underline the importance of developing an effective vaginal microbicide that would enable women to initiate measures to protect themselves.

Hence, the present invention is directed to compositions and methods for preventing HIV infections in a mammal or other animal, which include administering to the vagina or rectum of a mammal a therapeutically effective amount of a peptide of the present invention. Additional information on compositions and routes of administration is provided below.

### Recombinant Production of Peptides

Peptides of the invention can be made recombinantly using convenient vectors, expression systems and host cells. The invention therefore provides expression cassettes, vectors and host cells useful for expressing a peptide capable of inhibiting HIV fusion.

The expression cassettes of the invention include a promoter. Any promoter able to direct transcription of an encoded peptide or polypeptide may be used. Accordingly, many promoters may be included within the expression cassette of the invention. Some useful promoters include constitutive promoters, inducible promoters, regulated promoters, cell specific promoters, viral promoters, and synthetic promoters. A promoter is a nucleotide sequence that controls expression of an operably linked nucleic acid sequence by providing a recognition site for RNA polymerase, and possibly other factors, required for proper transcription. A promoter includes a minimal promoter, consisting only of all basal elements needed for transcription initiation, such as a TATA-box and/or other sequences that serve to specify the site of transcription initiation. A promoter may be obtained from a variety of different sources. For example, a promoter may be derived entirely from a native gene, be composed of different elements derived from different promoters found in nature, or be composed of nucleic acid sequences that are entirely synthetic. A promoter may be derived from many different types of organisms and tailored for use within a given cell.

For expression of a polypeptide in a bacterium, an expression cassette having a bacterial promoter will be used. A bacterial promoter is any DNA sequence capable of binding bacterial RNA polymerase and initiating the downstream (3") transcription of a coding sequence into mRNA. A promoter will have a transcription initiation region that is usually placed proximal to the 5' end of the coding sequence. This transcription initiation region usually includes an RNA polymerase binding site and a transcription initiation site. A second domain called an operator may be present and overlap an adjacent RNA polymerase binding site at which RNA synthesis begins. The operator permits negatively regulated (inducible) transcription, as a gene repressor protein may bind the operator and thereby inhibit transcription of a specific gene. Constitutive expression may occur in the absence of negative regulatory elements, such as the operator. In addition, positive regulation may be achieved by a gene activator protein binding sequence, which, if present is usually proximal (5') to the RNA polymerase binding sequence. An example of a gene activator protein is the catabolite activator protein (CAP), which helps initiate transcription of the *lac* operon in *E. coli* (Raibaud et al., Ann. Rev. Genet., 18:173 (1984)). Regulated expression may therefore be positive or negative, thereby either enhancing or reducing transcription.

Sequences encoding metabolic pathway enzymes provide particularly useful promoter sequences. Examples include promoter sequences derived from sugar metabolizing enzymes, such as galactose, lactose (*lac*) (Chang et al., Nature, 198:1056 (1977), and maltose. Additional examples include promoter sequences derived from biosynthetic enzymes such as tryptophan (Trp) (Goeddel et al., Nuc. Acids Res., 8:4057 (1980); Yelverton et al., Nuc. Acids Res., 9:731 (1981); U.S. Pat. No. 4,738,921; and EPO Publ. Nos. 036 776 and 121 775). The β-lactamase (bla) promoter system (Weissmann, "The cloning of interferon and other mistakes", in: Interferon 3 (ed. I. Gresser), 1981), and bacteriophage lambda P_{L} (Shimatake et al., Nature, 292:128 (1981)) and T5 (U.S. Pat. No. 4,689,406) promoter systems also provide useful promoter sequences. A preferred promoter is the Chlorella virus promoter (U.S. Patent No. 6,316,224).

Synthetic promoters that do not occur in nature also function as bacterial promoters. For example, transcription activation sequences of one bacterial or bacteriophage promoter may be joined with the operon sequences of another bacterial or bacteriophage promoter, creating a synthetic hybrid promoter (U.S. Pat. No. 4,551,433). For example, the *tac* promoter is a hybrid *trp-lac* promoter comprised of both *trp* promoter and *lac* operon sequences that is regulated by the *lac* repressor (Amann et al., Gene, 25:167 (1983); de Boer et al., Proc. Natl. Acad. Sci. USA, 80:21 (1983)). Furthermore, a bacterial promoter can include naturally occurring promoters of non-bacterial origin that have the ability to bind bacterial RNA polymerase and initiate transcription. A naturally occurring promoter of non-bacterial origin can also be coupled with a compatible RNA polymerase to produce high levels of expression of some genes in prokaryotes. The bacteriophage T7 RNA polymerase/promoter system is an example of a coupled promoter system (Studier et al., J. Mol. Biol., 189:113 (1986); Tabor et al., Proc. Natl. Acad. Sci. USA, 82:1074 (1985)). In addition, a hybrid promoter can also be comprised of a bacteriophage promoter and an *E. coli* operator region (EPO Publ. No. 267 851).

An expression cassette having a baculovirus promoter can be used for expression of a polypeptide in an insect cell. A baculovirus promoter is any DNA sequence capable of binding a baculovirus RNA polymerase and initiating transcription of a coding sequence into mRNA. A promoter will have a transcription initiation region that is usually placed proximal to the 5' end of the coding sequence. This transcription initiation region usually includes an RNA polymerase binding site and a transcription initiation site. A second domain called an enhancer may be present and is usually distal to the structural gene. A baculovirus promoter may be a regulated promoter or a constitutive promoter. Useful promoter sequences may be obtained from structural genes that are transcribed at times late in a viral infection cycle. Examples include sequences derived from the gene encoding the baculoviral polyhedron protein (Friesen et al., "The Regulation of Baculovirus Gene Expression", in: The Molecular Biology of Baculoviruses (ed. Walter Doerfler), 1986; and EPO Publ. Nos. 127 839 and 155 476) and the gene encoding the baculoviral p10 protein (Vlak et al., J. Gen. Virol., 69:765 (1988)).

Promoters that are functional in yeast are known to those of ordinary skill in the art. In addition to an RNA polymerase binding site and a transcription initiation site, a yeast promoter may also have a second region called an upstream activator sequence. The upstream activator sequence permits regulated expression that may be induced. Constitutive expression occurs in the absence of an upstream activator sequence. Regulated expression may be either positive or negative, thereby either enhancing or reducing transcription.

Promoters for use in yeast may be obtained from yeast genes that encode enzymes active in metabolic pathways. Examples of such genes include alcohol dehydrogenase (ADH) (EPO Publ. No. 284 044), enolase, glucokinase, glucose-6-phosphate isomerase, glyceraldehyde-3-phosphatedehydrogenase (GAP or GAPDH), hexokinase, phosphofructokinase, 3-phosphoglyceratemutase, and pyruvate kinase (PyK). (EPO Publ. No. 329 203). The yeast PHO5 gene, encoding acid phosphatase, also provides useful promoter sequences. (Myanohara et al., Proc. Natl. Acad. Sci. USA, 80:1 (1983)).

Synthetic promoters that do not occur in nature may also be used for expression in yeast. For example, upstream activator sequences from one yeast promoter may be joined with the transcription activation region of another yeast promoter, creating a synthetic hybrid promoter. Examples of such hybrid promoters include the ADH regulatory sequence linked to the GAP transcription activation region (U.S. Pat. Nos. 4,876,197 and 4,880,734). Other examples of hybrid promoters include promoters which consist of the regulatory sequences of either the ADH2, GAL4, GAL10, or PHO5 genes, combined with the transcriptional activation region of a glycolytic enzyme gene such as GAP or PyK (EPO Publ. No. 164 556). Furthermore, a yeast promoter can include naturally occurring promoters of non-yeast origin that have the ability to bind yeast RNA polymerase and initiate transcription. Examples of such promoters are known in the art. (Cohen et al., Proc. Natl. Acad. Sci. USA, 77:1078 (1980); Henikoff et al., Nature, 283:835 (1981); Hollenberg et al., Curr. Topics Microbiol. Immunol., 96:119 (1981)); Hollenberg et al., "The Expression of Bacterial Antibiotic Resistance Genes in the Yeast *Saccharomyces cerevisiae",* in: Plasmids of Medical, Environmental and Commercial Importance (eds. K. N. Timmis and A. Puhler), 1979; (Mercerau-Puigalon et al., Gene, 11:163 (1980); Panthier et al., Curr. Genet., 2:109 (1980)).

Many mammalian promoters are known in the art that may be used in conjunction with the expression cassette of the invention. Mammalian promoters often have a transcription initiating region, which is usually placed proximal to the 5' end of the coding sequence, and a TATA box, usually located 25-30 base pairs (bp) upstream of the transcription initiation site. The TATA box is thought to direct RNA polymerase II to begin RNA synthesis at the correct site. A mammalian promoter may also contain an upstream promoter element, usually located within 100 to 200 bp upstream of the TATA box. An upstream promoter element determines the rate at which transcription is initiated and can act in either orientation (Sambrook et al., "Expression of Cloned Genes in Mammalian Cells", in: Molecular Cloning: A Laboratory Manual, 2nd ed., 1989).

Mammalian viral genes are often highly expressed and have a broad host range; therefore sequences encoding mammalian viral genes often provide useful promoter sequences. Examples include the SV40 early promoter, mouse mammary tumour virus LTR promoter, adenovirus major late promoter (Ad MLP), and herpes simplex virus promoter. In addition, sequences derived from non-viral genes, such as the murine metallothionein gene, also provide useful promoter sequences. Expression may be either constitutive or regulated.

A mammalian promoter may also be associated with an enhancer. The presence of an enhancer will usually increase transcription from an associated promoter. An enhancer is a regulatory DNA sequence that can stimulate transcription up to 1000-fold when linked to homologous or heterologous promoters, with synthesis beginning at the normal RNA start site. Enhancers are active when they are placed upstream or downstream from the transcription initiation site, in either normal or flipped orientation, or at a distance of more than 1000 nucleotides from the promoter. (Maniatis et al., Science, 236:1237 (1987)); Alberts et al., Molecular Biology of the Cell, 2nd ed., 1989). Enhancer elements derived from viruses are often times useful, because they usually have a broad host range. Examples include the SV40 early gene enhancer (Dijkema et al., EMBO J., 4:761 (1985)) and the enhancer/promoters derived from the long terminal repeat (LTR) of the Rous Sarcoma Virus (Gorman et al., Proc. Natl. Acad. Sci. USA, 79:6777 (1982b)) and from human cytomegalovirus (Boshart et al., Cell, 41:521 (1985)). Additionally, some enhancers are regulatable and become active only in the presence of an inducer, such as a hormone or metal ion (Sassone-Corsi and Borelli, Trends Genet., 2:215 (1986); Maniatis et al., Science, 236:1237 (1987)).

It is understood that many promoters and associated regulatory elements may be used within the expression cassette of the invention to transcribe an encoded polypeptide. The promoters described above are provided merely as examples and are not to be considered as a complete list of promoters that are included within the scope of the invention.

The expression cassette of the invention may contain a nucleic acid sequence for increasing the translation efficiency of an mRNA encoding a polypeptide of the invention. Such increased translation serves to increase production of the polypeptide. The presence of an efficient ribosome binding site is useful for gene expression in prokaryotes. In bacterial mRNA, a conserved stretch of six nucleotides, the Shine-Dalgamo sequence, is usually found upstream of the initiating AUG codon. (Shine et al., Nature , 254:34 (1975)). This sequence is thought to promote ribosome binding to the mRNA by base pairing between the ribosome binding site and the 3' end of *Escherichia coli* 16S rRNA. (Steitz et al., "Genetic signals and nucleotide sequences in messenger RNA", in: Biological Regulation and Development: Gene Expression (ed. R. F. Goldberger), 1979)). Such a ribosome binding site, or operable derivatives thereof, are included within the expression cassette of the invention.

A translation initiation sequence can be derived from any expressed *Escherichia coli* gene and can be used within an expression cassette of the invention. Preferably the gene is a highly expressed gene. A translation initiation sequence can be obtained via standard recombinant methods, synthetic techniques, purification techniques, or combinations thereof, which are all well known. (Ausubel et al., Current Protocols in Molecular Biology, Green Publishing Associates and Wiley Interscience, NY. (1989); Beaucage and Caruthers, Tetra. Letts., 22:1859 (1981); VanDevanter et al., Nucleic Acids Res., 12:6159 (1984). Alternatively, translational start sequences can be obtained from numerous commercial vendors. (Operon Technologies; Life Technologies Inc, Gaithersburg, MD). In some embodiments, the T7 translation initiation sequence is used. The T7 translation initiation sequence is derived from the highly expressed T7 Gene 10 cistron and can have a sequence that includes TCTAGAAATAATTTTGTTTAACTTTAAGAAGGAGATATA (SEQ ID NO:20). Other examples of translation initiation sequences include, but are not limited to, the maltose-binding protein (Mal E gene) start sequence (Guan et al., Gene, 67:21 (1997)) present in the pMalc2 expression vector (New England Biolabs, Beverly, MA) and the translation initiation sequence for the following genes: thioredoxin gene (Novagen, Madison, WI), Glutathione-S-transferase gene (Pharmacia, Piscataway, NJ), β-galactosidase gene, chloramphenicol acetyltransferase gene and *E. coli* Trp E gene (Ausubel *et al.,* 1989, Current Protocols in Molecular Biology, Chapter 16, Green Publishing Associates and Wiley Interscience, NY).

Eucaryotic mRNA does not contain a Shine-Dalgarno sequence. Instead, the selection of the translational start codon is usually determined by its proximity to the cap at the 5' end of an mRNA. The nucleotides immediately surrounding the start codon in eucaryotic mRNA influence the efficiency of translation. Accordingly, one skilled in the art can determine what nucleic acid sequences will increase translation of a polypeptide encoded by the expression cassette of the invention. Such nucleic acid sequences are within the scope of the invention.

The invention therefore provides an expression cassette that includes a promoter operable in a selected host and a nucleic acid encoding a peptide having any one of SEQ ID NO:7-18. The expression cassette can have other elements, for example, termination signals, origins of replication, enhancers, and the like as described herein. The expression cassette can also be placed in a vector for easy replication and maintenance.

An expression cassette or nucleic acid construct of the invention is thought to be particularly advantageous for producing the peptides of the invention. Surprisingly, recombinant expression of the present peptides avoids degradation frequently observed for short peptides within a cell in which they are expressed. The present expression cassettes and nucleic acid constructs are also thought to be advantageous for producing the present peptides because these peptides are expressed and stored within inclusion bodies present within the host cells. Hence, the peptides can readily be purified from inclusion bodies.

In one embodiment, the recombinant peptides were expressed in *E. coli* strain BL21(DE3)/pLysS (Novagen). Cells were grown at 37 °C in LB media to an optical density of 0.8 at 600 nm and induced with isopropylthio-β-D-galactoside for 3-4 hr at 37 °C. The cells were centrifuged, frozen at -80°C, resuspended in 50 mM Tris-HCl (pH 8.0) and 1 mM EDTA plus 25% sucrose, and disrupted by sonication. Inclusion bodies of the cell lysate were isolated and washed three times with Triton buffer (20 mM Tris-HCl [pH 8.0], 1 mM EDTA, and 1% Triton X-100). The inclusion bodies were then solubilized in 50 mM Tris-HCl (pH 8.5) plus 8 M urea. Insoluble debris was removed by centrifugation (18,000 g, 1 hr, 4 °C); the supernatant was loaded on a DEAE Sepharose column (Amersham Pharmacia Biotech) equilibrated with buffer A (50 mM Tris-HCl [pH 8.5] plus 3 M urea). The soluble peptide was eluted with a linear salt gradient (0-500 mM NaCl in buffer A). The peptide solution was dialyzed into 5% acetic acid overnight at 4°C. Peptides from the soluble fraction were purified to homogeneity by reverse-phase high-performance liquid chromatography (Waters, Inc.) on a Vydac C-18 preparative column (Hesperia, CA), using a water-acetonitrile gradient in the presence of 0.1% trifluoroacetic acid, and lyophilized.

It has also been surprisingly found that the peptides of the invention are readily made and purified by the recombinant methods described herein. For ) example, isolation of the peptides is enhanced because the peptides are present in inclusion bodies that can readily be separated from other cellular components. Such inclusion bodies are more or less soluble under defined conditions that include, but are not limited to, pH, temperature, salt concentration, and protein concentration. Thus, an inclusion body can be insoluble in water but soluble in the presence of urea, acid, guanidinium chloride, and other agents. Hence, after recombinant expression of the present peptides in a host cell, the host cells can be isolated and lysed and inclusion bodies can be collected, for example, by centrifugation. The inclusion bodies can be rinsed with dilute buffer and then solubilized in urea or other agent. Insoluble debris can be removed by centrifugation and the solubilized peptides can be further purified, for example, by ion exchange chromatography or reverse-phase HPLC.

### Administration

The peptides of the invention, including their salts, are administered so as to achieve a reduction in at least one symptom associated with an infection, indication or disease, or a decrease in the amount of antibody associated with the indication or disease.

To achieve the desired effect(s), the peptide, a variant thereof or a combination thereof, may be administered as single or divided dosages, for example, of at least about 0.01 mg/kg to about 500 to 750 mg/kg, of at least about 0.01 mg/kg to about 300 to 500 mg/kg, at least about 0.1 mg/kg to about 100 to 300 mg/kg or at least about 1 mg/kg to about 50 to 100 mg/kg of body weight, although other dosages may provide beneficial results. The amount administered will vary depending on various factors including, but not limited to, the peptide chosen, the disease, the weight, the physical condition, the health, the age of the mammal, whether prevention or treatment is to be achieved, and if the peptide is chemically modified. Such factors can be readily determined by the clinician employing animal models or other test systems that are available in the art.

Administration of the therapeutic agents in accordance with the present invention may be in a single dose, in multiple doses, in a continuous or intermittent manner, depending, for example, upon the recipient's physiological condition, whether the purpose of the administration is therapeutic or prophylactic, and other factors known to skilled practitioners. The administration of the peptides of the invention may be essentially continuous over a pre-selected period of time or may be in a series of spaced doses. Both local and systemic administration is contemplated.

To prepare the composition, peptides are synthesized or otherwise obtained, purified as necessary or desired and then lyophilized and stabilized. The peptide can then be adjusted to the appropriate concentration, and optionally combined with other agents. The absolute weight of a given peptide included in a unit dose can vary widely. For example, about 0.01 to about 2 g, or about 0.1 to about 500 mg, of at least one peptide of the invention, or a plurality of peptides specific for a particular cell type can be administered. Alternatively, the unit dosage can vary from about 0.01 g to about 50 g, from about 0.01 g to about 35 g, from about 0.1 g to about 25 g, from about 0.5 g to about 12 g, from about 0.5 g to about 8 g, from about 0.5 g to about 4 g, or from about 0.5 g to about 2 g.

Daily doses of the peptides of the invention can vary as well. Such daily doses can range, for example, from about 0.1 g/day to about 50 g/day, from about 0.1 g/day to about 25 g/day, from about 0.1 g/day to about 12 g/day, from about 0.5 g/day to about 8 g/day, from about 0.5 g/day to about 4 g/day, and from about 0.5 g/day to about 2 g/day.

In the treatment or prevention of viral infections, an appropriate dosage level will generally be about 0.001 to 100 mg per kg patient body weight per day which can be administered in single or multiple doses. Preferably, the dosage level will be about 0.01 to about 25 mg/kg per day; more preferably about 0.05 to about 10 mg/kg per day. A suitable dosage level may be about 0.01 to 25 mg/kg per day, about 0.05 to 10 mg/kg per day, or about 0.1 to 5 mg/kg per day. Within this range the dosage may be about 0.005 to about 0.05, 0.05 to 0.5 or 0.5 to 5 mg/kg per day. For oral administration, the compositions are preferably provided in the form of tablets containing about 1 to 1000 milligrams of the active ingredient, particularly about 1, 5, 10, 15, 20, 25, 50, 75, 100, 150, 200, 250, 300, 400, 500, 600, 750, 800, 900, and 1000 milligrams of the active ingredient for the symptomatic adjustment of the dosage to the patient to be treated. The compounds may be administered on a regimen of 1 to 4 times per day, preferably once or twice per day.

Thus, one or more suitable unit dosage forms comprising the therapeutic peptides of the invention can be administered by a variety of routes including oral, parenteral (including subcutaneous, intravenous, intramuscular and intraperitoneal), rectal, vaginal, dermal, transdermal, intrathoracic, intrapulmonary and intranasal (respiratory) routes. The therapeutic peptides may also be formulated for sustained release (for example, using microencapsulation, see WO 94/ 07529, and U.S. Patent No.4,962,091). The formulations may, where appropriate, be conveniently presented in discrete unit dosage forms and may be prepared by any of the methods well known to the pharmaceutical arts. Such methods may include the step of mixing the therapeutic agent with liquid carriers, solid matrices, semi-solid carriers, finely divided solid carriers or combinations thereof, and then, if necessary, introducing or shaping the product into the desired delivery system.

When the therapeutic peptides of the invention are prepared for oral administration, they are generally combined with a pharmaceutically acceptable carrier, diluent or excipient to form a pharmaceutical formulation, or unit dosage form. For oral administration, the peptides may be present as a powder, a granular formulation, a solution, a suspension, an emulsion or in a natural or synthetic polymer or resin for ingestion of the active ingredients from a chewing gum. The active peptides may also be presented as a bolus, electuary or paste. Orally administered therapeutic peptides of the invention can also be formulated for sustained release, *e.g.*, the peptides can be coated, micro-encapsulated, or otherwise placed within a sustained delivery device. The total active ingredients in such formulations comprise from 0.1 to 99.9% by weight of the formulation.

By "pharmaceutically acceptable" it is meant a carrier, diluent, excipient, and/or salt that is compatible with the other ingredients of the formulation, and not deleterious to the recipient thereof.

Pharmaceutical formulations containing the therapeutic peptides of the invention can be prepared by procedures known in the art using well-known and readily available ingredients. For example, the peptide can be formulated with common excipients, diluents, or carriers, and formed into tablets, capsules, solutions, suspensions, powders, aerosols and the like. Examples of excipients, diluents, and carriers that are suitable for such formulations include buffers, as well as fillers and extenders such as starch, cellulose, sugars, mannitol, and silicic derivatives. Binding agents can also be included such as carboxymethyl cellulose, hydroxymethylcellulose, hydroxypropyl methylcellulose and other cellulose derivatives, alginates, gelatin, and polyvinyl-pyrrolidone. Moisturizing agents can be included such as glycerol, disintegrating agents such as calcium carbonate and sodium bicarbonate. Agents for retarding dissolution can also be included such as paraffin. Resorption accelerators such as quaternary ammonium compounds can also be included. Surface active agents such as cetyl alcohol and glycerol monostearate can be included. Adsorptive carriers such as kaolin and bentonite can be added. Lubricants such as talc, calcium and magnesium stearate, and solid polyethyl glycols can also be included. Preservatives may also be added. The compositions of the invention can also contain thickening agents such as cellulose and/or cellulose derivatives. They can also contain gums such as xanthan, guar or carbo gum or gum arabic, or alternatively polyethylene glycols, bentones and montmorillonites, and the like.

For example, tablets or caplets containing the peptides of the invention can include buffering agents such as calcium carbonate, magnesium oxide and magnesium carbonate. Caplets and tablets can also include inactive ingredients such as cellulose, pre-gelatinized starch, silicon dioxide, hydroxy propyl methyl cellulose, magnesium stearate, microcrystalline cellulose, starch, talc, titanium dioxide, benzoic acid, citric acid, com starch, mineral oil, polypropylene glycol, sodium phosphate, zinc stearate, and the like. Hard or soft gelatin capsules containing at least one peptide of the invention can contain inactive ingredients such as gelatin, microcrystalline cellulose, sodium lauryl sulfate, starch, talc, and titanium dioxide, and the like, as well as liquid vehicles such as polyethylene glycols (PEGs) and vegetable oil. Moreover, enteric-coated caplets or tablets containing one or more peptides of the invention are designed to resist disintegration in the stomach and dissolve in the more neutral to alkaline environment of the duodenum.

The therapeutic peptides of the invention can also be formulated as elixirs or solutions for convenient oral administration or as solutions appropriate for parenteral administration, for instance by intramuscular, subcutaneous, intraperitoneal or intravenous routes. The pharmaceutical formulations of the therapeutic peptides of the invention can also take the form of an aqueous or anhydrous solution or dispersion, or alternatively the form of an emulsion or suspension or salve.

Thus, the therapeutic peptides may be formulated for parenteral administration (*e*.*g*., by injection, for example, bolus injection or continuous infusion) and may be presented in unit dose form in ampoules, pre- filled syringes, small volume infusion containers or in multi-dose containers. As noted above, preservatives can be added to help maintain the shelve life of the dosage form. The active peptides and other ingredients may form suspensions, solutions, or emulsions in oily or aqueous vehicles, and may contain formulatory agents such as suspending, stabilizing and/or dispersing agents. Alternatively, the active peptides and other ingredients may be in powder form, obtained by aseptic isolation of sterile solid or by lyophilization from solution, for constitution with a suitable vehicle, *e.g.*, sterile, pyrogen-free water, before use.

These formulations can contain pharmaceutically acceptable carriers, vehicles and adjuvants that are well known in the art. It is possible, for example, to prepare solutions using one or more organic solvent(s) that is/are acceptable from the physiological standpoint, chosen, in addition to water, from solvents such as acetone, ethanol, isopropyl alcohol, glycol ethers such as the products sold under the name "Dowanol," polyglycols and polyethylene glycols, C₁-C₄ alkyl esters of short-chain acids, ethyl or isopropyl lactate, fatty acid triglycerides such as the products marketed under the name "Miglyol," isopropyl myristate, animal, mineral and vegetable oils and polysiloxanes.

It is possible to add, if necessary, an adjuvant chosen from antioxidants, surfactants, other preservatives, film-forming, keratolytic or comedolytic agents, perfumes, flavorings and colorings. Antioxidants such as t-butylhydroquinone, butylated hydroxyanisole, butylated hydroxytoluene and α-tocopherol and its derivatives can be added.

Also contemplated are combination products that include one or more peptides of the present invention and one or more other anti-viral or antimicrobial agents.

Additionally, the peptides are well suited to formulation as sustained release dosage forms and the like. The formulations can be so constituted that they release the active peptide, for example, in a particular part of the intestinal or respiratory tract or within the vagina or rectum, possibly over a period of time. Coatings, envelopes, and protective matrices may be made, for example, from polymeric substances, such as polylactide-glycolates, liposomes, microemulsions, microparticles, nanoparticles, or waxes. These coatings, envelopes, and protective matrices are useful to coat indwelling devices, *e*.*g*., stents, catheters, peritoneal dialysis tubing, draining devices and the like.

For topical, vaginal or rectal administration, the therapeutic agents may be formulated as is known in the art for direct application to a target area. Forms chiefly conditioned for topical application take the form, for example, of creams, milks, gels, foams, dispersion or microemulsions, lotions thickened to a greater or lesser extent, impregnated pads of tampons, ointments or sticks, aerosol formulations (*e.g*., sprays or foams), soaps, detergents, lotions or cakes of soap. Other conventional forms for this purpose include wound dressings, coated bandages or other polymer coverings, ointments, creams, foams, lotions, pastes, jellies, sprays, and aerosols. Thus, the therapeutic peptides of the invention can be delivered via patches or bandages for dermal administration. Alternatively, the peptide can be formulated to be part of an adhesive polymer, such as polyacrylate or acrylate/vinyl acetate copolymer. For long-term applications it might be desirable to use microporous and/or breathable backing laminates, so hydration or maceration of the skin can be minimized. The backing layer can be any appropriate thickness that will provide the desired protective and support functions. A suitable thickness will generally be from about 10 to about 200 microns.

Ointments and creams may, for example, be formulated with an aqueous or oily base with the addition of suitable thickening and/or gelling agents. Lotions may be formulated with an aqueous or oily base and will in general also contain one or more emulsifying agents, stabilizing agents, dispersing agents, suspending agents, thickening agents, or coloring agents. The active peptides can also be delivered via iontophoresis, e.g., as disclosed in U.S. Patent Nos. 4,140,122; 4,383,529; or 4,051,842. The percent by weight of a therapeutic agent of the invention present in a topical formulation will depend on various factors, but generally will be from 0.01 % to 95% of the total weight of the formulation, and typically 0.1-85% by weight

Drops, such as eye drops or nose drops, may be formulated with one or more of the therapeutic peptides in an aqueous or non-aqueous base also comprising one or more dispersing agents, solubilizing agents or suspending agents. Liquid sprays are conveniently delivered from pressurized packs. Drops can be delivered via a simple eye dropper-capped bottle, or via a plastic bottle adapted to deliver liquid contents dropwise, via a specially shaped closure.

The therapeutic peptide may further be formulated for topical administration in the mouth or throat. For example, the active ingredients may be formulated as a lozenge further comprising a flavored base, usually sucrose and acacia or tragacanth; pastilles comprising the composition in an inert base such as gelatin and glycerin or sucrose and acacia; and mouthwashes comprising the composition of the present invention in a suitable liquid carrier.

The pharmaceutical formulations of the present invention may include, as optional ingredients, pharmaceutically acceptable carriers, diluents, solubilizing or emulsifying agents, and salts of the type that are available in the art. Examples of such substances include normal saline solutions such as physiologically buffered saline solutions and water. Specific non-limiting examples of the carriers and/or diluents that are useful in the pharmaceutical formulations of the present invention include water and physiologically acceptable buffered saline solutions such as phosphate buffered saline solutions pH 7.0-8.0.

The peptides of the invention can also be administered to the respiratory tract. Thus, the present invention also provides aerosol pharmaceutical formulations and dosage forms for use in the methods of the invention. In general, such dosage forms comprise an amount of at least one of the agents of the invention effective to treat or prevent the clinical symptoms of a specific infection, indication or disease. Any statistically significant attenuation of one or more symptoms of an infection, indication or disease that has been treated pursuant to the method of the present invention is considered to be a treatment of such infection, indication or disease within the scope of the invention.

Alternatively, for administration by inhalation or insufflation, the composition may take the form of a dry powder, for example, a powder mix of the therapeutic agent and a suitable powder base such as lactose or starch. The powder composition may be presented in unit dosage form in, for example, capsules or cartridges, or, *e.g*., gelatin or blister packs from which the powder may be administered with the aid of an inhalator, insufflator, or a metered-dose inhaler (see, for example, the pressurized metered dose inhaler (MDI) and the dry powder inhaler disclosed in Newman, S. P. in Aerosols and the Lung, Clarke, S. W. and Davia, D. eds., pp. 197-224, Butterworths, London, England, 1984).

Therapeutic peptides of the present invention can be administered as a dry powder or in an aqueous solution when administered in an aerosol or inhaled form. Other aerosol pharmaceutical formulations may comprise, for example, a physiologically acceptable buffered saline solution containing between about 0.1 mg/ml and about 100 mg/ml of one or more of the peptides of the present invention specific for the indication or disease to be treated. Dry aerosol in the form of finely divided solid peptide or nucleic acid particles that are not dissolved or suspended in a liquid are also useful in the practice of the present invention. Peptides of the present invention may be formulated as dusting powders and comprise finely divided particles having an average particle size of between about 1 and 5 µm, alternatively between 2 and 3 µm. Finely divided particles may be prepared by pulverization and screen filtration using techniques well known in the art. The particles may be administered by inhaling a predetermined quantity of the finely divided material, which can be in the form of a powder. It will be appreciated that the unit content of active ingredient or ingredients contained in an individual aerosol dose of each dosage form need not in itself constitute an effective amount for treating the particular infection, indication or disease since the necessary effective amount can be reached by administration of a plurality of dosage units. Moreover, the effective amount may be achieved using less than the dose in the dosage form, either individually, or in a series of administrations.

For administration to the upper (nasal) or lower respiratory tract by inhalation, the therapeutic peptides of the invention are conveniently delivered from a nebulizer or a pressurized pack or other convenient means of delivering an aerosol spray. Pressurized packs may comprise a suitable propellant such as dichlorodifluoromethane, trichlorofluoromethane, dichlorotetrafluoroethane, carbon dioxide or other suitable gas. In the case of a pressurized aerosol, the dosage unit may be determined by providing a valve to deliver a metered amount. Nebulizers include, but are not limited to, those described in U.S. Patent Nos. 4,624,251; 3,703,173; 3,561,444; and 4,635,627. Aerosol delivery systems of the type disclosed herein are available from numerous commercial sources including Fisons Corporation (Bedford, Mass.), Schering Corp. (Kenilworth, NJ) and American Pharmoseal Co., (Valencia, CA). For intra-nasal administration, the therapeutic agent may be administered via nose drops, a liquid spray, such as via a plastic bottle atomizer or metered-dose inhaler. Typical of atomizers are the Mistometer (Wintrop) and the Medihaler (Riker).

Furthermore, the active ingredients may also be used in combination with other therapeutic agents, for example, pain relievers, anti-inflammatory agents, anti-bacterial agents, antihistamines, bronchodilators and the like, whether for the conditions described or some other condition.

The present invention further pertains to a packaged pharmaceutical composition for controlling viral infections such as a kit or other container. The kit or container holds a therapeutically effective amount of a pharmaceutical composition for controlling viral infections and instructions for using the pharmaceutical composition for control of the viral infection. The pharmaceutical composition includes at least one peptide of the present invention, in a therapeutically effective amount such that viral infection is controlled.

The invention is further illustrated by the following non-limiting Example.

### EXAMPLE: Stable Peptide Inhibitors of HIV Fusion

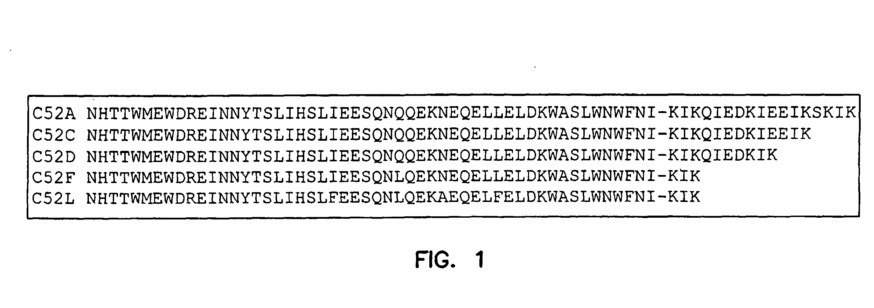

This Example describes the design of more potent analogues of C-peptide inhibitors by systematic mutagenesis and modeling of recombinant peptide sequences in order to augment interhelical packing and other the factors involved in stabilization of their three dimensional structures. The peptides of the invention were designed to have 3 to 17 amino acid α-helical stabilizing peptides at the carboxyl terminus of the C52 peptide fragment of HIV-1 gp41 (Figure 1). Four point mutations were introduced into the C52F peptide to enhance its potential for forming an α-helix. The peptides so formed were referred to as the C52A, C52C, C52D, C52F and C52L peptide inhibitors.

### Materials and Methods

### Gene construction and Protein production

Nucleic acids encoding gp41 (residues 624-675, numbered according to their position in gp160 of the HXB2 HIV-1 strain) and the N-terminal 17 amino acids of GCN4-pII (Harbury et al., 1994) were synthesized by using optimal codons for *Escherichia coli* expression (Chen et al., 1994). This nucleic acid construct was inserted into the *Nde* I and *Bam* HI restriction sites of pET-24a (Novagen) to yield pC52A (see Figure 1). Plasmids expressing the C52C, C52D, C52F, and C52L peptides were derived from pC52A by single-stranded mutagenesis (Kunkel et al., 1987). The sequences of the C52C, C52D, C52F, and C52L peptides are shown in Table 4. All coding sequences were confirmed by DNA sequencing.

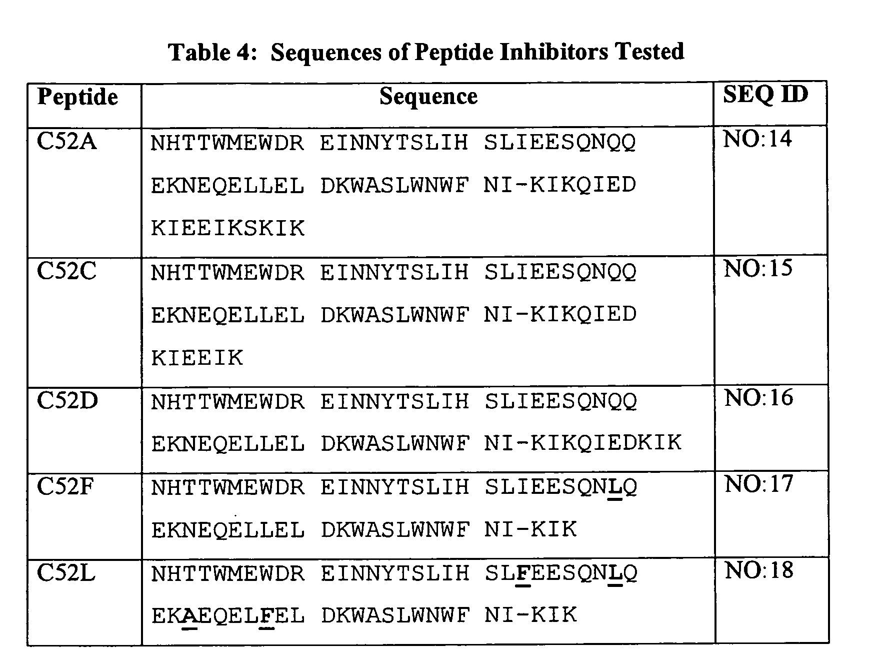

Additional synthetic peptides were synthesized as control peptides. For example, the T-20 peptide was synthesized that peptide has the sequence provided below (SEQ ID NO:6).

Acetyl-YTSLIHSLIE ESQNQQEKNE QELLELDKWA SLWNWF

Another control peptide employed is the C34 peptide, with the following sequence (SEQ ID NO: 19): WMEWDREINN YTSLIHSLIE ESQNQQEKNE QELL Synthetic peptides called C peptides that are derived from the gp41 HR2 that are like the C34 peptide and the T-20 peptide have been observed to inhibit HIV-1 entry (Jiang et al., 1993; Wild et al., 1994). Hence, the C34 and T -20 peptides act as positive controls that can inhibit HIV fusion to some degree in the assays employed herein.

The recombinant peptides were expressed in *E*. *coli* strain BL21(DE3)/pLysS (Novagen). Cells were grown at 37 °C in LB media to an optical density of 0.8 at 600 nm and induced with isopropylthio-β-D-galactoside for 3-4 hr at 37 °C. The cells were centrifuged, frozen at -80°C, resuspended in 50 mM Tris-HCl (pH 8.0) and 1 mM EDTA plus 25% sucrose, and disrupted by sonication. Inclusion bodies of the cell lysate were isolated and washed three times with Triton buffer (20 mM Tris-HCl [pH 8.0], 1 mM EDTA, and 1% Triton X-100). The inclusion bodies were then solubilized in 50 mM Tris-HCl (pH 8.5) plus 8 M urea. Insoluble debris was removed by centrifugation (18,000 x g, 1 hr, 4°C); the supernatant was loaded on a DEAE Sepharose column (Amersham Pharmacia Biotech) equilibrated with buffer A (50 mM Tris-HCl [pH 8.5] plus 3 M urea). The soluble peptide was eluted with a linear salt gradient (0-500 mM NaCl in buffer A). The peptide solution was dialyzed into 5% acetic acid overnight at 4°C. Peptides from the soluble fraction were purified to homogeneity by reverse-phase high-performance liquid chromatography (Waters, Inc.) on a Vydac C-18 preparative column (Hesperia, CA), using a water-acetonitrile gradient in the presence of 0.1% trifluoroacetic acid, and lyophilized. The molecular weights of each peptide were confirmed by using matrix-assisted laser desorption ionization-time-of-flight mass spectrometry (PerSeptive Biosystems, Framingham, MA).

### Circular Dichroism Spectroscopy

CD measurements of the peptides were carried out on an Aviv 62 DS circular dichroism spectrometer (Aviv Biomedical, Lakewood, NJ). Peptides were dissolved in 50 mM Tris-HCl (pH 8.0) and 10 mM NaCl with a total peptide concentration of 10 µM. The wavelength dependence of molar ellipticity, [θ], was monitored at 4 °C as the average of five scans, using a five-second integration time at 1.0 nm wavelength increments. Spectra were baseline-corrected against the cuvette with buffer alone. Helix content was estimated from the CD signal by dividing the mean residue ellipticity at 222 nm by the value expected for 100% helix formation by helices of comparable size, - 34,000 deg.cm² dmol⁻¹ (Chen et al., 1974). Thermal stability was determined by monitoring the change in CD signal at 222 nm as a function of temperature, and thermal melts were performed in two-degree intervals with a two -minute equilibration at the desired temperature, and an integration time of 30 s. Reversibility was checked by repeated scans.

*Assay for HIV Fusion.* Peptides were tested for inhibition of cell-cell fusion between Chinese hamster ovary (CHO) cells expressing the HXB2 HIV-1 envelope glycoprotein [K. Kozarsky, M. Penman, L. Basiripour, W. Haseltine, J. Sodroski, M. Krieger, *J. Acquir. Immune Defic. Syndr.* **2*,*** 163 (1989)] and HeLa-CD4-LTR-Beta-gal cells (M. Emerman, NIH AIDS Research and Reference Reagent Program). In this assay, syncytia formation was quantified by the selective expression of a beta-galactosidase gene in fused cells [J. Kimpton and M. Emerman, *J. Virol.* **66**, 2232, (1992)]. An estimated 2 x 10⁴ CHO cells and 4 x 10⁴ HeLa cells were mixed in wells of a 48 -well dish and co-cultured for 24-48 hr at 37°C in the presence of varying concentrations of peptide inhibitor. After the monolayers were stained with the colorimetric substrate 5-bromo-4-chloro-3-indolyl-β-D-galactoside, syncytia containing three or more nuclei were counted. IC₅₀ values were estimated by fitting inhibition data to a Langmuir equation: y = k/(1 + [peptide]/IC₅₀), where y = number of syncytia, and k is a scaling constant.

*PBMC Infectivity Assay.* PBMC were isolated from healthy blood donors by Ficoll-Hypaque centrifugation and then stimulated for 2-3 days with phytohemagglutinin (5 µg/ml) and interleukin-2 (100 U/ml). The inhibition assay was performed essentially as described previously (Trkola, A., T. Ketas, V. N. KewalRamani, F. Endorf, J. M. Binley, H. Katinger, J. Robinson, D. R. Littman, and J. P. Moore. 1998. Neutralization sensitivity of human immunodeficiency virus type 1 primary isolates to antibodies and CD4-based reagents is independent of co-receptor usage. J. Virol. 72:1876-1885; Trkola, A., W. A. Paxton, S. P. Monard, J. A. Hoxie, M. A. Siani, D. A. Thompson, L. Wu, C. R. Mackay, R. Horuk, and J. P. Moore. 1998. Genetic subtype-independent inhibition of human immunodeficiency virus type 1 replication by CC and CXC chemokines. J. Virol. 72:396-404).

Briefly, the virus inoculum was adjusted to 400-1,000 TCID₅₀/ml in assay medium (RPMI 1640, 10% FCS, 100 U of interleukin-2 per ml, glutamine, and peptides), and 50 µl aliquots were incubated with serial dilutions of the peptide inhibitors (50 µl) for 1 h at 37 °C. The calculated 50% inhibition doses (ID₅₀s) refer to the concentrations of the peptides in this pre-incubation mixture. RBMC (4 x 10⁵ in 100 µl of medium) were then added. The final concentration of virus in the cultures was 20-50 TCID₅₀/well, corresponding to 100-250 TCID₅₀/ml. Peptides, virus, and cells were then incubated for 5-7 days at 37 °C, and the extent of viral replication was determined by p24 antigen ELISA o f the culture supernatants. The viral inocula in 50% tissue culture infectious doses (TCID₅₀) were 400-1000/ml.

### Results

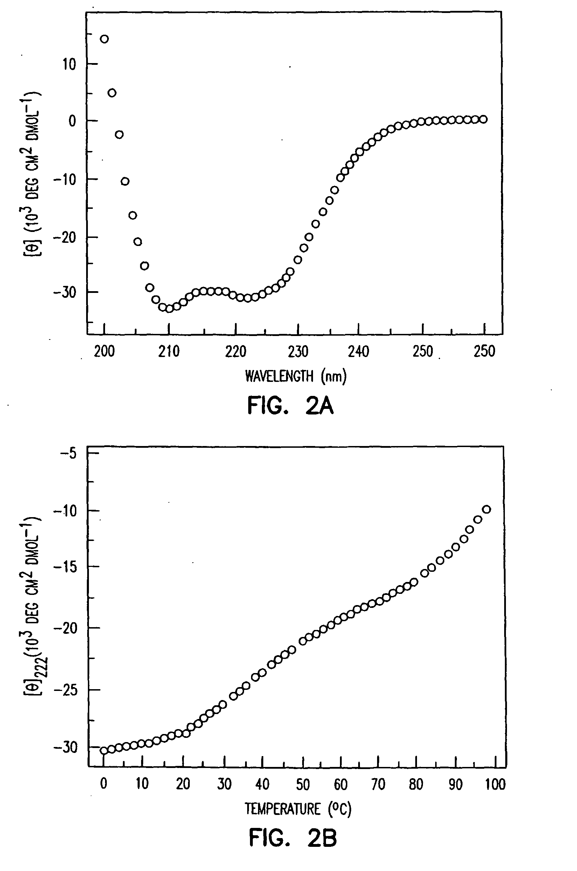

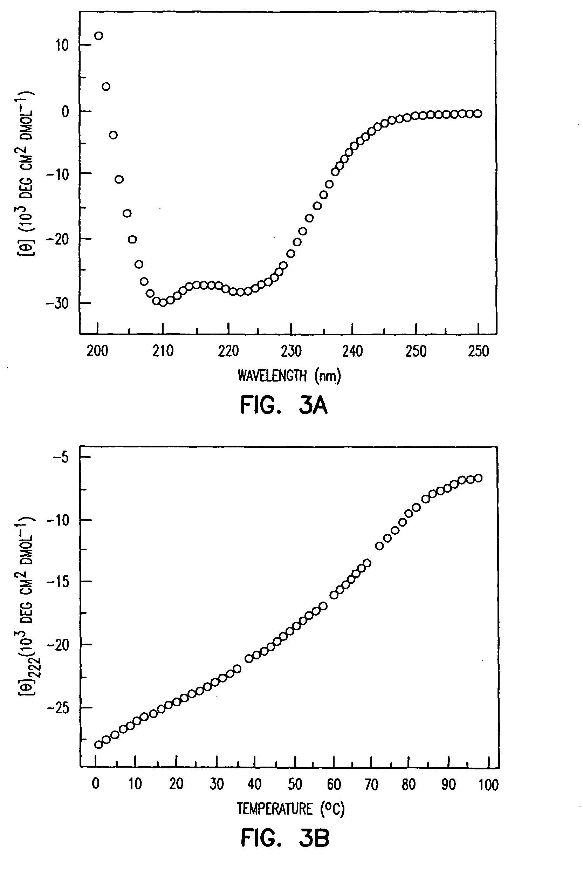

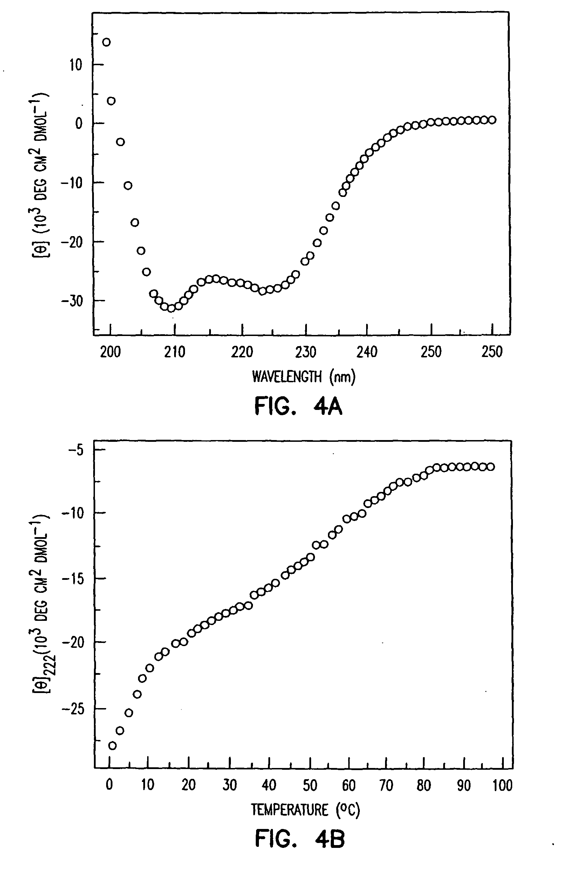

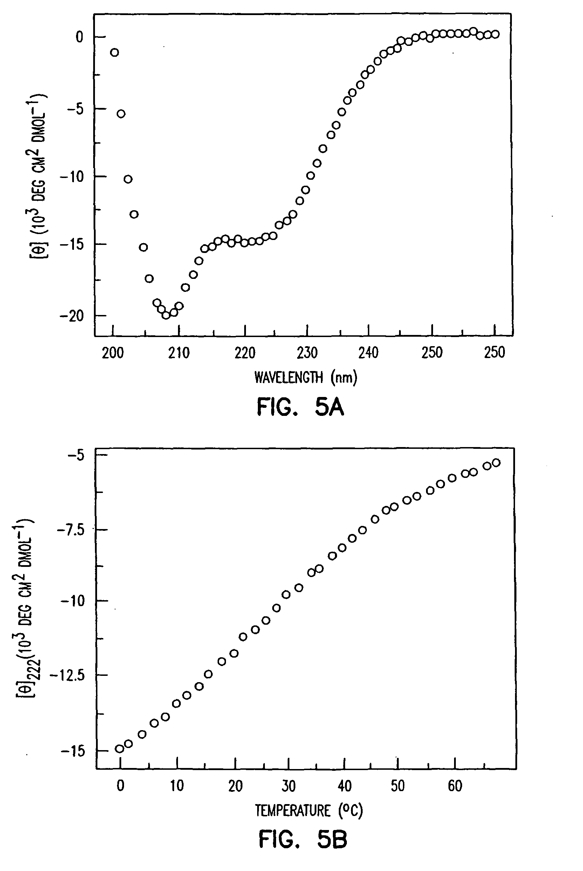

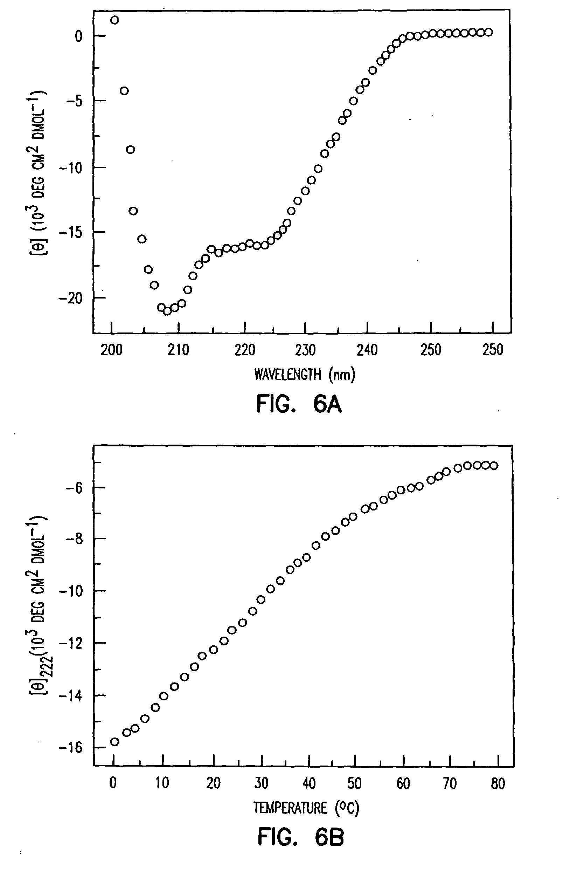

The C52L, C52A, C52C, C52D, and C52F peptides were produced by bacterial expression and purified by reverse-phase high-performance liquid chromatography as described above. Circular dichroism experiments indicate that each of the C52L, C52A, C52C, C52D, and C52F peptides forms an α-helical structure in solution (Figures 2-6).

As shown in Table 5, the C52A, C52C, C52D, and C52F peptides were able to inhibit HIV-1 fusion *in vitro* at lower concentrations than the C34 and T-20 peptides that acted as positive controls.

**Table 5:**

| **Peptide Inhibition of HIV-1 Cell-Cell Fusion** | | |
|---|---|---|
| **Peptide** | **IC**_{**50**} **(nM)** | **SEQ ID NO:** |
| C34 | 26 | 19 |
| T-20 | 33 | 6 |
| C52A | 18 | 14 |
| C52C | 8 | 15 |
| C52D | 5 | 16 |
| C52F | 10 | 17 |
| C52L | 8 | 18 |

Two separate PBMC infectivity assays were performed for most of the HIV strains. These results are shown in Table 6. As shown in Table 6, the C52F and C52L peptides were able to inhibit HIV-1 infection *in vitro* at lower concentrations than the C34 and T-20 peptides that acted as positive controls.

**Table 6:**

| **Antiviral Activity of Peptide Inhibitors Human PBMC Infectivity Assays** | | | | | | | |
|---|---|---|---|---|---|---|---|
| **IC**_{**50**} **(nM)** | | | | | | | |
| **Virus isolate** | **C34** | **T-20** | **C52A** | **C52C** | **C52D** | **C52F** | **C52L** |
| HIV-1 JFRL | 15 | 10 | | | | 5 | 6 |
| | 89 | 9 | 89 | 199 | 34 | | |
| HIV-1 DJ258 | 7 | 18 | | | | 9 | 11 |
| | 158 | 83 | 1034 | 225 | 166 | | |
| HIV-1 NL4-3 | 10 | 44 | | | | 23 | 20 |
| | 37 | 35 | 347 | 83 | 75 | | |
| HIV-1 Case C | 28 | 10 | | | | 49 | 17 |
| 7/86 | 258 | 4 | 288 | 261 | 1 | | |
| HIV-1 94ZW103 | 3 | 18 | | | | 4 | 3 |
| | 37 | 13 | 166 | 38 | 34 | | |
| HIV-1 UG270 | 48 | 139 | | | | 68 | 61 |
| | 254 | 257 | 856 | 206 | 239 | | |
| HIV-1 CM.235 | 9 | 14 | | | | 11 | 12 |
| | 115 | 79 | 129 | 201 | 171 | | |
| HIV-1 BZ162 | 2 | 13 | | | | 2 | 3 |
| | 12 | 28 | 174 | 38 | 28 | | |

### References

Chen, Y. H., Yang, J. T., and Chau, K. H. (1974) *Biochemistry 13,* 3350-3359.

Chen, G.Q., Choi, I., Ramachandran, B. & Gouaux, J.E. (1994) J. Am. Chem. Soc. 116,8799-8800.

Harbury, P. B., Kim, P. S. & Alber, T. (1994). Crystal structure of an isoleucine-zipper trimer. *Nature,* **371**, 80-83.

Kunkel, T. A., Roberts, J. D. & Zakour, R. A. (1987). Rapid and efficient site-specific mutagenesis without phenotypic selection. *Methods Enzymol.* **154**, 367-382.

All patents and publications referenced or mentioned herein are indicative of the levels of skill of those skilled in the art to which the invention pertains, and each such referenced patent or publication is hereby incorporated by reference to the same extent as if it had been incorporated by reference in its entirety individually or set forth herein in its entirety. Applicants reserve the right to physically incorporate into this specification any and all materials and information from any such cited patents or publications.

The specific methods and compositions described herein are representative of preferred embodiments and are exemplary and not intended as limitations on the scope of the invention. Other objects, aspects, and embodiments will occur to those skilled in the art upon consideration of this specification, and are encompassed within the spirit of the invention as defined by the scope of the claims. It will be readily apparent to one skilled in the art that varying substitutions and modifications may be made to the invention disclosed herein without departing from the scope and spirit of the invention. The invention illustratively described herein suitably may be practiced in the absence of any element or elements, or limitation or limitations, which is not specifically disclosed herein as essential. The methods and processes illustratively described herein suitably may be practiced in differing orders of steps, and that they are not necessarily restricted to the orders of steps indicated herein or in the claims. As used herein and in the appended claims, the singular forms "a," "an," and ''the'' include plural reference unless the context clearly dictates otherwise. Thus, for example, a reference to "a host cell" includes a plurality (for example, a culture or population) of such host cells, and so forth. Under no circumstances may the patent be interpreted to be limited to the specific examples or embodiments or methods specifically disclosed herein. Under no circumstances may the patent be interpreted to be limited by any statement made by any Examiner or any other official or employee of the Patent and Trademark Office unless such statement is specifically and without qualification or reservation expressly adopted in a responsive writing by Applicants.

The terms and expressions that have been employed are used as terms of description and not of limitation, and there is no intent in the use of such terms and expressions to exclude any equivalent of the features shown and described or portions thereof, but it is recognized that various modifications are possible within the scope of the invention as claimed. Thus, it will be understood that although the present invention has been specifically disclosed by preferred embodiments and optional features, modification and variation of the concepts herein disclosed may be resorted to by those skilled in the art, and that such modifications and variations are considered to be within the scope of this invention as defined by the appended claims.

The invention has been described broadly and generically herein. Each of the narrower species and subgeneric groupings falling within the generic disclosure also form part of the invention. This includes the generic description of the invention with a proviso or negative limitation removing any subject matter from the genus, regardless of whether or not the excised material is specifically recited herein.

Other embodiments are within the following claims. In addition, where features or aspects of the invention are described in terms of Markush groups, those skilled in the art will recognize that the invention is also thereby described in terms of any individual member or subgroup of members of the Markush group.

## Claims

1. A stabilizing peptide that when linked to a second peptide or polypeptide can confer increased stability to said second peptide or polypeptide, wherein said stabilizing peptide comprises any one of the amino acid sequences of SEQ ID NOs: 10, 11, 12, or 13 or a variant or derivative thereof

2. A stabilizing peptide according to claim 1 wherein the stabilizing peptide can enhance the propensity of the second peptide or polypeptide to fold into an α-helix.

3. A peptide inhibitor that can inhibit fusion of a human immunodeficiency virus with a mammalian cell, wherein the peptide inhibitor is linked to a stabilizing peptide according to claim 1 or 2 .

4. The peptide inhibitor of claim 3, wherein the peptide inhibitor comprises any one of the amino acid sequences of SEQ ID NOs: 6, 7, 8, or 9 or a variant or derivative thereof.

5. The peptide inhibitor of claim 3 or 4, wherein the peptide inhibitor comprises any one of the amino acid sequences of SEQ ID NOs: 14, 15, 16, 17, or 18 or variants or derivatives thereof.

6. The peptide inhibitor of any one of claims 3-5, wherein the peptide inhibitor can bind to a gp41 subunit of the envelope glycoprotein.

7. The peptide inhibitor of claim 6, wherein the peptide inhibitor binds to a coiled coil of a prehairpin intermediate formed by the gp41 subunit of the envelope glycoprotein.

8. A composition comprising a carrier and a stabilizing peptide according to claim 1 or 2, or a peptide inhibitor comprising any one of the amino acid sequences of SEQ ID NOs: 7, 8, 9, 14, 15, 16, 17 or 18 or a variant or derivative thereof.

9. A composition according to claim 8 wherein the stabilising peptide is linked to a peptide inhibitor that can inhibit fusion of a human immunodeficiency virus

10. A composition according to claim 9 wherein the linked peptide inhibitor comprises any one of amino acid sequences of SEQ ID NOs: 6, 7, 8 or 9.

11. A composition according to any one of claims 8-10 wherein the peptide can bind to a gp41 subunit of the envelope glycoprotein.

12. A peptide inhibitor according to any one of claims 3-7 or comprising any one of the amino acid sequences of SEQ ID NOs: 7, 8 or 9 or a variant or derivative thereof for use in therapy.

13. A peptide inhibitor according to claim 12 for use in preventing or treating infection by human immunodeficiency virus in a mammal.

14. A method of making a stable peptide, which method comprises expressing in a host cell a stable peptide comprising a first segment comprising any one of the amino acid sequences of SEQ ID NOs: 10, 11, 12 or 13, and a second segment comprising a selected amino acid sequence, wherein the stable peptide is sufficiently stable to be isolated intact from the host cell.

15. A method of making a stable peptide inhibitor that can inhibit fusion of a human immunodeficiency virus with mammalian cells, which method comprises expressing in a host cell a peptide inhibitor comprising any one of the amino acid sequences of SEQ ID NOs: 8, 9, 14, 15, 16, 17, or 18, wherein the peptide inhibitor is sufficiently stable to be isolated intact from the host cell.

16. The method of claim 12 or 13, wherein the host cell is a bacterial host cell.


SEQUENCE LISTING

<110> Cornell Research Foundation, Inc.

<120> Stabilizing Peptides

<130> 59.68.83921

<150> US 60/492,704
<151> 2003-08-05

<150> US
<151> 2003-05-23

<160> 20

<170> FastSEQ for Windows Version 4.0

<210> 1
<211> 748
<212> PRT
<213> Human immunodeficiency virus 1 (HIV-1)

<400> 1
Met Arg Val Lys Glu Lys Tyr Gln His Leu Arg Arg Trp Gly Trp Arg
 1               5                  10                  15
Trp Gly Thr Met Leu Leu Gly Met Leu Met Ile Cys Ser Ala Thr Glu
            20                  25                  30
Lys Leu Trp Val Thr Val Tyr Tyr Gly Val Pro Val Trp Lys Glu Ala
        35                  40                  45
Thr Thr Thr Leu Phe Cys Ala Ser Asp Ala Lys Ala Tyr Asp Thr Glu
    50                  55                  60
Val His Asn Val Trp Ala Thr His Ala Cys Val Pro Thr Asp Pro Asn
65                  70                  75                  80
Pro Gln Glu Val Val Leu Val Asn Val Thr Glu Asn Phe Asn Met Trp
                85                  90                  95
Lys Asn Asp Met Val Glu Gln Met His Glu Asp Ile Ile Ser Leu Trp
            100                 105                 110
Asp Gln Ser Leu Lys Pro Cys Val Lys Leu Thr Pro Leu Cys Val Ser
        115                 120                 125
Leu Lys Cys Thr Asp Leu Lys Asn Asp Thr Asn Thr Asn Ser Ser Ser

    130                 135                 140
Gly Gly Met Ile Met Glu Lys Gly Glu Ile Lys Asn Cys Ser Phe Asn
145                 150                 155                 160
Ile Ser Thr Ser Ile Arg Gly Lys Val Gln Lys Glu Tyr Ala Phe Phe
                165                 170                 175
Tyr Lys Leu Asp Ile Ile Pro Ile Asp Asn Asp Thr Thr Ser Tyr Thr
            180                 185                 190
Leu Thr Ser Cys Asn Thr Ser Val Ile Thr Gln Ala Cys Pro Lys Val
        195                 200                 205
Ser Phe Glu Pro Ile Pro Ile His Tyr Cys Ala Pro Ala Gly Phe Ala
    210                 215                 220
Ile Leu Lys Cys Asn Asn Lys Thr Phe Asn Gly Thr Gly Pro Cys Thr
225                 230                 235                 240
Asn Val Ser Thr Val Gln Cys Thr His Gly Ile Arg Pro Val Val Ser
                245                 250                 255
Thr Gln Leu Leu Leu Asn Gly Ser Leu Ala Glu Glu Glu Val Val Ile
            260                 265                 270
Arg Ser Ala Asn Phe Thr Asp Asn Val Lys Thr Ile Ile Val Gln Leu
        275                 280                 285
Asn Gln Ser Val Glu Ile Asn Cys Thr Lys Pro Asn Asn Asn Thr Gly
    290                 295                 300
Lys Arg Ile Arg Ile Gln Arg Gly Pro Gly Arg Thr Phe Val Thr Ile
305                 310                 315                 320
Gly Lys Ile Gly Asn Met Arg Gln Ala His Cys Asn Ile Ser Arg Ala
                325                 330                 335
Lys Trp Asn Asn Thr Leu Lys Gln Ile Ala Ser Lys Leu Arg Glu Gln
            340                 345                 350
Tyr Gly Asn Asn Lys Thr Ile Ile Phe Lys Gln Ser Ser Gly Gly Asp
        355                 360                 365
Leu Glu Ile Val Thr His Ser Phe Asn Cys Gly Gly Glu Phe Phe Tyr
    370                 375                 380
Cys Asn Ser Thr Gln Leu Phe Asn Ser Thr Trp Phe Asn Ser Thr Gly
385                 390                 395                 400
Ser Asn Asn Thr Glu Gly Ser Asp Thr Ile Thr Leu Pro Cys Arg Ile
                405                 410                 415
Lys Gln Ile Ile Asn Met Trp Gln Glu Val Gly Lys Ala Met Tyr Ala
            420                 425                 430
Pro Pro Ile Ser Gly Gln Ile Arg Cys Ser Ser Asn Ile Thr Gly Leu
        435                 440                 445
Leu Leu Thr Arg Asp Gly Gly Asn Asn Asn Asn Gly Ser Glu Ile Phe
    450                 455                 460

Arg Pro Gly Gly Gly Asp Met Arg Asp Asn Trp Arg Ser Glu Leu Tyr
465                 470                 475                 480
Lys Tyr Lys Val Val Lys Ile Glu Pro Leu Gly Val Ala Pro Thr Lys
                485                 490                 495
Ala Lys Arg Arg Val Val Gln Arg Glu Lys Arg Ala Val Gly Ile Gly
            500                 505                 510
Ala Leu Phe Leu Gly Phe Leu Gly Ala Ala Gly Ser Thr Met Gly Ala
        515                 520                 525
Ala Ser Met Thr Leu Thr Val Gln Ala Arg Gln Leu Leu Ser Gly Ile
    530                 535                 540
Val Gln Gln Gln Asn Asn Leu Leu Arg Ala Ile Glu Ala Gln Gln His
545                 550                 555                 560
Leu Leu Gln Leu Thr Val Trp Gly Ile Lys Gln Leu Gln Ala Arg Ile
                565                 570                 575
Leu Ala Val Glu Arg Tyr Leu Lys Asp Gln Gln Leu Leu Gly Ile Trp
            580                 585                 590
Gly Cys Ser Gly Lys Leu Ile Cys Thr Thr Ala Val Pro Trp Asn Ala
        595                 600                 605
Ser Trp Ser Asn Lys Ser Leu Glu Arg Ile Trp Asn His Thr Thr Trp
    610                 615                 620
Met Glu Trp Asp Arg Glu Ile Asn Asn Tyr Thr Ser Leu Ile His Ser
625                 630                 635                 640
Leu Ile Glu Glu Ser Gln Asn Gln Gln Glu Lys Asn Glu Gln Glu Leu
                645                 650                 655
Leu Glu Leu Asp Lys Trp Ala Ser Leu Trp Asn Trp Phe Asn Ile Thr
            660                 665                 670
Asn Trp Leu Trp Tyr Val Lys Ile Phe Ile Met Ile Val Gly Gly Leu
        675                 680                 685
Val Gly Leu Arg Ile Val Phe Ala Val Leu Ser Ile Val Asn Arg Val
    690                 695                 700
Arg Gln Gly Tyr Ser Pro Leu Ser Phe Gln Thr His Leu Pro Thr Pro
705                 710                 715                 720
Gly Gly Pro Asp Arg Pro Glu Gly Ile Glu Glu Glu Gly Gly Glu Arg
                725                 730                 735
Asp Arg Asp Arg Ser Ile Arg Leu Val Asn Gly Ser
            740                 745

<210> 2
<211> 358
<212> PRT
<213> Human immunodeficiency virus 1 (HIV-1)

<220>
<221> SITE
<222> 349
<223> Xaa = any amino acid.

<400> 2
Lys Ala Lys Arg Arg Val Val Gln Arg Glu Lys Arg Ala Val Gly Met
1               5                   10                  15
Gly Ala Ala Phe Phe Leu Gly Phe Leu Gly Ala Ala Gly Ser Thr Met
            20                  25                  30
Gly Ala Ala Ser Leu Thr Leu Thr Val Gln Ala Arg Leu Leu Leu Ser
        35                  40                  45
Gly Ile Val Gln Gln Gln Asn Asn Leu Leu Arg Ala Ile Glu Ala His
    50                  55                  60
Glu His Leu Leu Gln Leu Thr Val Trp Gly Ile Lys Gln Leu Gln Ala
65                  70                  75                  80
Arg Ile Leu Ala Val Glu Arg Tyr Leu Lys Asp Gln Gln Leu Leu Gly
                85                  90                  95
Ile Trp Gly Cys Ser Gly Lys Leu Ile Cys Thr Thr Thr Val Pro Trp
            100                 105                 110
Asn Ala Ser Trp Ser Asn Lys Ser Leu Asp Lys Ile Trp Asn Asn Met
        115                 120                 125
Thr Trp Met Glu Trp Asp Arg Glu Ile Asn Asn Tyr Thr Ser Leu Ile
    130                 135                 140
Tyr Thr Leu Ile Glu Gln Ser Gln Asn Gln Gln Glu Lys Asn Glu Gln
145                 150                 155                 160
Glu Leu Leu Glu Leu Asp Lys Trp Ala Ser Leu Trp Asn Trp Phe Asp
                165                 170                 175
Ile Thr Gln Trp Leu Trp Tyr Ile Lys Ile Phe Ile Met Ile Val Gly
            180                 185                 190
Gly Leu Ile Gly Leu Arg Ile Val Phe Thr Val Leu Ser Ile Val Asn
        195                 200                 205
Arg Val Arg Gln Gly Tyr Ser Pro Leu Ser Phe Gln Thr Arg Arg Pro
    210                 215                 220

Ala Arg Arg Gly Pro Asp Arg Pro Glu Gly Ile Glu Glu Glu Gly Gly
225                 230                 235                 240
Glu Arg Asp Arg Asp Arg Ser Gly Arg Leu Val Asn Gly Phe Leu Ala
                245                 250                 255
Leu Ile Trp Asp Asp Leu Arg Ser Leu Cys Leu Phe Ser Tyr His Arg
            260                 265                 270
Leu Arg Asp Leu Leu Leu Ile Val Thr Arg Ile Val Glu Leu Leu Gly
        275                 280                 285
Arg Arg Gly Trp Glu Val Leu Lys Tyr Leu Trp Asn Leu Leu Gln Tyr
    290                 295                 300
Trp Ser Gln Glu Leu Lys Asn Ser Ala Val Ser Leu Leu Asn Ala Thr
305                 310                 315                 320
Ala Ile Ala Val Ala Glu Gly Thr Asp Arg Val Ile Glu Leu Leu Gln
                325                 330                 335
Arg Ala Phe Arg Ala Ile Leu His Ile Pro Arg Arg Xaa Arg Gln Gly
            340                 345                 350
Leu Glu Arg Ala Leu Leu
        355

<210> 3
<211> 117
<212> PRT
<213> Human immunodeficiency virus 1 (HIV-1)

<400> 3
Ala Gln Gln His Leu Leu Arg Leu Thr Val Trp Gly Ile Lys Gln Leu
 1               5                  10                  15
Gln Ala Arg Val Leu Ala Leu Glu Arg Tyr Leu Lys Asp Gln Gln Leu
            20                  25                  30
Leu Gly Ile Trp Gly Cys Ser Gly Arg Leu Ile Cys Thr Thr Asn Val
        35                  40                  45
Pro Trp Asn Ser Ser Trp Ser Asn Lys Thr Tyr Asn Asp Ile Trp Asp
    50                  55                  60
Asn Met Thr Trp Leu Gln Trp Asp Lys Glu Ile Ser Asn Tyr Thr Asn
65                  70                  75                  80
Ile Ile Tyr Thr Leu Ile Glu Glu Ser Gln Asn Gln Gln Glu Lys Asn
                85                  90                  95
Glu Gln Asp Leu Leu Ala Leu Asp Lys Trp Ala Ser Leu Trp Ser Trp
            100                 105                 110
Phe Asp Ile Thr Asn

        115

<210> 4
<211> 117
<212> PRT
<213> Human immunodeficiency virus 1 (HIV-1)

<400> 4
Arg Ser Gln His Leu Leu Lys Leu Thr Val Trp Gly Ile Lys Gln Leu
1               5                   10                  15
Gln Ala Arg Val Leu Ala Leu Glu Arg Tyr Leu Lys Asp Gln Gln Leu
            20                  25                  30
Leu Gly Ile Trp Gly Cys Ser Gly Lys Leu Ile Cys Thr Thr Asn Val
        35                  40                  45
Pro Trp Asn Ser Ser Trp Ser Asn Lys Thr Tyr Glu Tyr Ile Trp Gly
    50                  55                  60
Asn Met Thr Trp Leu Gln Trp Glu Lys Glu Ile Asp Asn Tyr Thr Ser
65                  70                  75                  80
Leu Ile Tyr Thr Leu Ile Glu Glu Ser Gln Ile Gln Gln Glu Lys Asn
                85                  90                  95
Glu Gln Asp Leu Leu Ala Leu Asp Glu Trp Ala Ser Leu Trp Ser Trp
            100                 105                 110
Phe Ser Ile Thr Lys
        115

<210> 5
<211> 856
<212> PRT
<213> Human immunodeficiency virus type 1 (HXB2 ISOLATE)

<400> 5
Met Arg Val Lys Glu Lys Tyr Gln His Leu Trp Arg Trp Gly Trp Arg
1               5                   10                  15
Trp Gly Thr Met Leu Leu Gly Met Leu Met Ile Cys Ser Ala Thr Glu
            20                  25                  30
Lys Leu Trp Val Thr Val Tyr Tyr Gly Val Pro Val Trp Lys Glu Ala
        35                  40                  45

Thr Thr Thr Leu Phe Cys Ala Ser Asp Ala Lys Ala Tyr Asp Thr Glu
    50                  55                  60
Val His Asn Val Trp Ala Thr His Ala Cys Val Pro Thr Asp Pro Asn
65                  70                  75                  80
Pro Gln Glu Val Val Leu Val Asn Val Thr Glu Asn Phe Asn Met Trp
                85                  90                  95
Lys Asn Asp Met Val Glu Gln Met His Glu Asp Ile Ile Ser Leu Trp
            100                 105                 110
Asp Gln Ser Leu Lys Pro Cys Val Lys Leu Thr Pro Leu Cys Val Ser
        115                 120                 125
Leu Lys Cys Thr Asp Leu Lys Asn Asp Thr Asn Thr Asn Ser Ser Ser
    130                 135                 140
Gly Arg Met Ile Met Glu Lys Gly Glu Ile Lys Asn Cys Ser Phe Asn
145                 150                 155                 160
Ile Ser Thr Ser Ile Arg Gly Lys Val Gln Lys Glu Tyr Ala Phe Phe
                165                 170                 175
Tyr Lys Leu Asp Ile Ile Pro Ile Asp Asn Asp Thr Thr Ser Tyr Lys
            180                 185                 190
Leu Thr Ser Cys Asn Thr Ser Val Ile Thr Gln Ala Cys Pro Lys Val
        195                 200                 205
Ser Phe Glu Pro Ile Pro Ile His Tyr Cys Ala Pro Ala Gly Phe Ala
    210                 215                 220
Ile Leu Lys Cys Asn Asn Lys Thr Phe Asn Gly Thr Gly Pro Cys Thr
225                 230                 235                 240
Asn Val Ser Thr Val Gln Cys Thr His Gly Ile Arg Pro Val Val Ser
                245                 250                 255
Thr Gln Leu Leu Leu Asn Gly Ser Leu Ala Glu Glu Glu Val Val Ile
            260                 265                 270
Arg Ser Val Asn Phe Thr Asp Asn Ala Lys Thr Ile Ile Val Gln Leu
        275                 280                 285
Asn Thr Ser Val Glu Ile Asn Cys Thr Arg Pro Asn Asn Asn Thr Arg
    290                 295                 300
Lys Arg Ile Arg Ile Gln Arg Gly Pro Gly Arg Ala Phe Val Thr Ile
305                 310                 315                 320
Gly Lys Ile Gly Asn Met Arg Gln Ala His Cys Asn Ile Ser Arg Ala
                325                 330                 335
Lys Trp Asn Asn Thr Leu Lys Gln Ile Ala Ser Lys Leu Arg Glu Gln
            340                 345                 350
Phe Gly Asn Asn Lys Thr Ile Ile Phe Lys Gln Ser Ser Gly Gly Asp
        355                 360                 365
Pro Glu Ile Val Thr His Ser Phe Asn Cys Gly Gly Glu Phe Phe Tyr

370                 375                 380
Cys Asn Ser Thr Gln Leu Phe Asn Ser Thr Trp Phe Asn Ser Thr Trp
385                 390                 395                 400
Ser Thr Glu Gly Ser Asn Asn Thr Glu Gly Ser Asp Thr Ile Thr Leu
                405                 410                 415
Pro Cys Arg Ile Lys Gln Ile Ile Asn Met Trp Gln Lys Val Gly Lys
            420                 425                 430
Ala Met Tyr Ala Pro Pro Ile Ser Gly Gln Ile Arg Cys Ser Ser Asn
        435                 440                 445
Ile Thr Gly Leu Leu Leu Thr Arg Asp Gly Gly Asn Ser Asn Asn Glu
    450                 455                 460
Ser Glu Ile Phe Arg Pro Gly Gly Gly Asp Met Arg Asp Asn Trp Arg
465                 470                 475                 480
Ser Glu Leu Tyr Lys Tyr Lys Val Val Lys Ile Glu Pro Leu Gly Val
                485                 490                 495
Ala Pro Thr Lys Ala Lys Arg Arg Val Val Gln Arg Glu Lys Arg Ala
            500                 505                 510
Val Gly Ile Gly Ala Leu Phe Leu Gly Phe Leu Gly Ala Ala Gly Ser
        515                 520                 525
Thr Met Gly Ala Ala Ser Met Thr Leu Thr Val Gln Ala Arg Gln Leu
    530                 535                 540
Leu Ser Gly Ile Val Gln Gln Gln Asn Asn Leu Leu Arg Ala Ile Glu
545                 550                 555                 560
Ala Gln Gln His Leu Leu Gln Leu Thr Val Trp Gly Ile Lys Gln Leu
                565                 570                 575
Gln Ala Arg Ile Leu Ala Val Glu Arg Tyr Leu Lys Asp Gln Gln Leu
            580                 585                 590
Leu Gly Ile Trp Gly Cys Ser Gly Lys Leu Ile Cys Thr Thr Ala Val
        595                 600                 605
Pro Trp Asn Ala Ser Trp Ser Asn Lys Ser Leu Glu Gln Ile Trp Asn
    610                 615                 620
His Thr Thr Trp Met Glu Trp Asp Arg Glu Ile Asn Asn Tyr Thr Ser
625                 630                 635                 640
Leu Ile His Ser Leu Ile Glu Glu Ser Gln Asn Gln Gln Glu Lys Asn
                645                 650                 655
Glu Gln Glu Leu Leu Glu Leu Asp Lys Trp Ala Ser Leu Trp Asn Trp
            660                 665                 670
Phe Asn Ile Thr Asn Trp Leu Trp Tyr Ile Lys Leu Phe Ile Met Ile
        675                 680                 685
Val Gly Gly Leu Val Gly Leu Arg Ile Val Phe Ala Val Leu Ser Ile
    690                 695                 700

Val Asn Arg Val Arg Gln Gly Tyr Ser Pro Leu Ser Phe Gln Thr His
705                 710                 715                 720
Leu Pro Thr Pro Arg Gly Pro Asp Arg Pro Glu Gly Ile Glu Glu Glu
                725                 730                 735
Gly Gly Glu Arg Asp Arg Asp Arg Ser Ile Arg Leu Val Asn Gly Ser
            740                 745                 750
Leu Ala Leu Ile Trp Asp Asp Leu Arg Ser Leu Cys Leu Phe Ser Tyr
        755                 760                 765
His Arg Leu Arg Asp Leu Leu Leu Ile Val Thr Arg Ile Val Glu Leu
    770                 775                 780
Leu Gly Arg Arg Gly Trp Glu Ala Leu Lys Tyr Trp Trp Asn Leu Leu
785                 790                 795                 800
Gln Tyr Trp Ser Gln Glu Leu Lys Asn Ser Ala Val Ser Leu Leu Asn
                805                 810                 815
Ala Thr Ala Ile Ala Val Ala Glu Gly Thr Asp Arg Val Ile Glu Val
            820                 825                 830
Val Gln Gly Ala Cys Arg Ala Ile Arg His Ile Pro Arg Arg Ile Arg
        835                 840                 845
Gln Gly Leu Glu Arg Ile Leu Leu
    850                 855

<210> 6
<211> 36
<212> PRT
<213> Artificial Sequence

<220>
<223> A synthetic construct.

<400> 6
Tyr Thr Ser Leu Ile His Ser Leu Ile Glu Glu Ser Gln Asn Gln Gln
1               5                   10                  15
Glu Lys Asn Glu Gln Glu Leu Leu Glu Leu Asp Lys Trp Ala Ser Leu
            20                  25                  30
Trp Asn Trp Phe
        35

<210> 7
<211> 52

<212> PRT
<213> Artificial Sequence

<220>
<223> A synthetic construct.

<400> 7
Asn His Thr Thr Trp Met Glu Trp Asp Arg Glu Ile Asn Asn Tyr Thr
1               5                   10                  15
Ser Leu Ile His Ser Leu Ile Glu Glu Ser Gln Asn Gln Gln Glu Lys
            20                  25                  30
Asn Glu Gln Glu Leu Leu Glu Leu Asp Lys Trp Ala Ser Leu Trp Asn
        35                  40                  45
Trp Phe Asn Ile
    50

<210> 8
<211> 52
<212> PRT
<213> Artificial Sequence

<220>
<223> A synthetic construct.

<400> 8
Asn His Thr Thr Trp Met Glu Trp Asp Arg Glu Ile Asn Asn Tyr Thr
1               5                   10                  15
Ser Leu Ile His Ser Leu Ile Glu Glu Ser Gln Asn Leu Gln Glu Lys
            20                  25                  30
Asn Glu Gln Glu Leu Leu Glu Leu Asp Lys Trp Ala Ser Leu Trp Asn
        35                  40                  45
Trp Phe Asn Ile
    50

<210> 9
<211> 52
<212> PRT
<213> Artificial Sequence

<220>
<223> A synthetic construct.

<400> 9
Asn His Thr Thr Trp Met Glu Trp Asp Arg Glu Ile Asn Asn Tyr Thr
1               5                   10                  15
Ser Leu Ile His Ser Leu Phe Glu Glu Ser Gln Asn Leu Gln Glu Lys
            20                  25                  30
Ala Glu Gln Glu Leu Phe Glu Leu Asp Lys Trp Ala Ser Leu Trp Asn
        35                  40                  45
Trp Phe Asn Ile
    50

<210> 10
<211> 3
<212> PRT
<213> Artificial Sequence

<220>
<223> A synthetic construct.

<400> 10
Lys Ile Lys
1

<210> 11
<211> 10
<212> PRT
<213> Artificial Sequence

<220>
<223> A synthetic construct.

<400> 11
Lys Ile Lys Gln Ile Glu Asp Lys Ile Lys
1               5                   10

<210> 12

<211> 13
<212> PRT
<213> Artificial Sequence

<220>
<223> A synthetic construct.

<400> 12
Lys Ile Lys Gln Ile Glu Asp Lys Ile Glu Glu Ile Lys
1               5                   10

<210> 13
<211> 17
<212> PRT
<213> Artificial Sequence

<220>
<223> A synthetic construct.

<400> 13
Lys Ile Lys Gln Ile Glu Asp Lys Ile Glu Glu Ile Lys Ser Lys Ile
1               5                   10                  15
Lys

<210> 14
<211> 69
<212> PRT
<213> Artificial Sequence

<220>
<223> A synthetic construct.

<400> 14
Asn His Thr Thr Trp Met Glu Trp Asp Arg Glu Ile Asn Asn Tyr Thr
1               5                   10                  15
Ser Leu Ile His Ser Leu Ile Glu Glu Ser Gln Asn Gln Gln Glu Lys
            20                  25                  30
Asn Glu Gln Glu Leu Leu Glu Leu Asp Lys Trp Ala Ser Leu Trp Asn

35                  40                  45
Trp Phe Asn Ile Lys Ile Lys Gln Ile Glu Asp Lys Ile Glu Glu Ile
    50                  55                  60
Lys Ser Lys Ile Lys
65

<210> 15
<211> 65
<212> PRT
<213> Artificial Sequence

<220>
<223> A synthetic construct.

<400> 15
Asn His Thr Thr Trp Met Glu Trp Asp Arg Glu Ile Asn Asn Tyr Thr
 1               5                  10                  15
Ser Leu Ile His Ser Leu Ile Glu Glu Ser Gln Asn Gln Gln Glu Lys
            20                  25                  30
Asn Glu Gln Glu Leu Leu Glu Leu Asp Lys Trp Ala Ser Leu Trp Asn
        35                  40                  45
Trp Phe Asn Ile Lys Ile Lys Gln Ile Glu Asp Lys Ile Glu Glu Ile
    50                  55                  60
Lys
65

<210> 16
<211> 62
<212> PRT
<213> Artificial Sequence

<220>
<223> A synthetic construct.

<400> 16
Asn His Thr Thr Trp Met Glu Trp Asp Arg Glu Ile Asn Asn Tyr Thr
 1               5                  10                  15
Ser Leu Ile His Ser Leu Ile Glu Glu Ser Gln Asn Gln Gln Glu Lys
            20                  25                  30

Asn Glu Gln Glu Leu Leu Glu Leu Asp Lys Trp Ala Ser Leu Trp Asn
        35                  40                  45
Trp Phe Asn Ile Lys Ile Lys Gln Ile Glu Asp Lys Ile Lys
    50                  55                  60

<210> 17
<211> 55
<212> PRT
<213> Artificial Sequence

<220>
<223> A synthetic construct.

<400> 17
Asn His Thr Thr Trp Met Glu Trp Asp Arg Glu Ile Asn Asn Tyr Thr
 1               5                  10                  15
Ser Leu Ile His Ser Leu Ile Glu Glu Ser Gln Asn Leu Gln Glu Lys
            20                  25                  30
Asn Glu Gln Glu Leu Leu Glu Leu Asp Lys Trp Ala Ser Leu Trp Asn
        35                  40                  45
Trp Phe Asn Ile Lys Ile Lys
    50                  55

<210> 18
<211> 55
<212> PRT
<213> Artificial Sequence

<220>
<223> A synthetic construct.

<400> 18
Asn His Thr Thr Trp Met Glu Trp Asp Arg Glu Ile Asn Asn Tyr Thr
 1               5                  10                  15
Ser Leu Ile His Ser Leu Phe Glu Glu Ser Gln Asn Leu Gln Glu Lys
            20                  25                  30
Ala Glu Gln Glu Leu Phe Glu Leu Asp Lys Trp Ala Ser Leu Trp Asn
        35                  40                  45
Trp Phe Asn Ile Lys Ile Lys

50                  55

<210> 19
<211> 34
<212> PRT
<213> Artificial Sequence

<220>
<223> A synthetic construct.

<400> 19
Trp Met Glu Trp Asp Arg Glu Ile Asn Asn Tyr Thr Ser Leu Ile His
1               5                   10                  15
Ser Leu Ile Glu Glu Ser Gln Asn Gln Gln Glu Lys Asn Glu Gln Glu
            20                  25                  30
Leu Leu

<210> 20
<211> 39
<212> DNA
<213> Artificial Sequence

<220>
<223> A synthetic construct.

<400> 20
tctagaaata attttgttta actttaagaa ggagatata                              39